# EUROPEAN PATENT SPECIFICATION

(11) **EP 4 344 703 B1**
(45) Date of publication and mention of the grant of the patent: **18.11.2026**
(21) Application number: 23196616.9
(22) Date of filing: 10.06.2016
(51) Int. Cl.: A61K 31/4709, A61K 9/00, A61K 31/496, A61P 7/06, C07D 215/36

(54) **A PYRUVATE KINASE ACTIVATOR FOR TREATING THALASSEMIA, SICKLE CELL ANEMIA OR PYROVATE KINASE DEFICIENCY**
PYRUVATKINASEAKTIVATOR ZUR BEHANDLUNG VON THALASSÄMIE, SICHELZELLANÄMIE ODER PYROVATKINASEMANGEL
ACTIVATEUR DE PYRUVATE KINASE POUR LE TRAITEMENT DE LA THALASSÉMIE, DE LA DRÉPANOCYTOSE OU D'UNE DÉFICIENCE EN PYROVATE KINASE

(30) Priority: 11.06.2015 US 201562174216 P
(43) Date of publication of application: 03.04.2024
(62) Document number(s) of the earlier application(s) in accordance with Art. 76 EPC: 16808374.9 / 3 307 271
(73) Proprietor: Agios Pharmaceuticals, Inc., Cambridge, MA 02139 (US)
(72) Inventor: AGRESTA, Samuel, V., Lexington, MA (US); CHEN, Yue, Quincy, MA (US); COHEN, Marvin Barry, Newtown, PA (US); DANG, Leonard Luan C., Boston, MA (US); KUNG, Charles, Arlington, MA (US); MERICA, Elizabeth A., Boston, MA (US); SILVER, Bruce Alan, Dunkirk, MD (US); YANG, Hua, Acton, MA (US)
(74) Representative: Maiwald GmbH

(56) References cited:
- WO-A1-2012/151451
- US-A1- 2014 155 408
- US-A1- 2014 323 467
- KUNG CHARLES ET AL: "AG-348 Activation of Pyruvate Kinase in Vivo Enhances Red Cell Glycolysis in Mice", BIOSIS, BIOSCIENCES INFORMATION SERVICE, PHILADELPHIA, PA, US, 1 December 2014 (2014-12-01), XP002787328
- DATABASE BIOSIS [online] BIOSCIENCES INFORMATION SERVICE, PHILADELPHIA, PA, US; December 2014 (2014-12-01), YANG HUA ET AL: "Phase I Single (SAD) and Multiple Ascending Dose (MAD) Studies of the Safety, Tolerability, Pharmacokinetics (PK) and Pharmacodynamics (PD) of AG-348, a First-in-Class Allosteric Activator of Pyruvate Kinase-R, in Healthy Subjects", XP002787329, Database accession no. PREV201500280858
- DIMITRIOS ANASTASIOU: "Pyruvate kinase M2 activators promote tetramer formation and suppress tumorigenesis Correspondence: performed in vivo pharmacology and ADME studies wrote the paper with significant input from Y", NAT CHEM BIOL., vol. 8, no. 10, 1 October 2012 (2012-10-01), pages 839 - 847, XP055333200

## Description

### CLAIM OF PRIORITY

This application claims priority from U.S.S.N. 62/174,216 filed June 11, 2015.

### BACKGROUND

Pyruvate kinase deficiency (PKD) is one of the most common enzyme defects in erythrocytes in humans due to autosomal recessive mutations of the *PKLR* gene (Zanella, A., et al., Br J Haematol 2005, 130 (1), 11-25). It is also the most frequent enzyme mutation in the central glycolytic pathway and only second to glucose-6 phosphate dehydrogenase (G6PD) deficiency (Kedar, P., et al., Clin Genet 2009, 75 (2), 157-62) of the hexose monophosphate shunt.

Human erythrocytes are unique in that they anucleate when mature. Immature erythocytes have nuclei but during early erythropoiesis prior to becoming circulating reticulocytes they extrude nuclei as well as other organelles such as mitochondria, endoplasmic reticulum, and Golgi apparatus, in order to make room for oxygen-carrying hemoglobin. As a result of lacking mitochondria, mature red blood cells do not utilize any of the oxygen they transport to economically synthesize adenosine triphosphate (ATP) as other normal differentiated cells do. Instead, red blood cells depend entirely on anaerobic glycolysis to cycle nicotinamide adenine dinucleotide (NAD⁺) and to make ATP, an essential energy source largely used to drive ATPase-dependent K⁺/Na⁺ and Ca²⁺ pumps, in order to maintain cell membrane integrity and pliability as they navigate through blood vessels. In PKD disorder, two major distinctive metabolic abnormalities are ATP depletion and concomitant increase of 2,3-diphosphoglycerate consistent with accumulation of upper glycolytic intermediates. Moreover, one of the consequences of decreased ATP and pyruvate levels is lowered lactate level leading to inability to regenerate NAD⁺ through lactate dehydrogenase for further use in glycolysis. The lack of ATP disturbs the cation gradient across the red cell membrane, causing the loss of potassium and water, which causes cell dehydration, contraction, and crenation, and leads to premature destruction and diminished lifetime of the red blood cells (RBCs). Such defective RBCs are destroyed in the spleen, and excessive hemolysis rate in the spleen leads to the manifestation of hemolytic anemia. The exact mechanism by which PKD sequesters newly matured RBCs in the spleen to effectively shorten overall half-lives of circulating RBCs is not yet clear, but recent studies suggest that metabolic dysregulation affects not only cell survival but also the maturation process resulting in ineffective erythropoiesis (Aizawa, S. et al., Exp Hematol 2005, 33 (11), 1292-8).

Pyruvate kinase catalyzes the transfer of a phosphoryl group from phosphoenolpyruvate (PEP) to ADP, yielding one molecule of pyruvate and one molecule of ATP. The enzyme has an absolute requirement for Mg²⁺ and K⁺ cations to drive catalysis. PK functions as the last critical step in glycolysis because it is an essentially irreversible reaction under physiological conditions. In addition to its role of synthesizing one of the two ATP molecules from the metabolism of glucose to pyruvate, pyruvate kinase is also an important cellular metabolism regulator. It controls the carbon flux in lower-glycolysis to provide key metabolite intermediates to feed biosynthetic processes, such as pentose-phosphate pathway among others, in maintaining healthy cellular metabolism. Because of these critical functions, pyruvate kinase is tightly controlled at both gene expression and enzymatic allostere levels. In mammals, fully activated pyruvate kinase exists as a tetrameric enzyme. Four different isozymes (M1, M2, L and R) are expressed from two separate genes. Erythrocyte-specific isozyme PKR is expressed from the *PKLR* gene ("L gene") located on chromosome 1q21. This same gene also encodes the PKL isozyme, which is predominately expressed in the liver. *PKLR* consists of 12 exons with exon 1 is erythroid-specific whereas exon 2 is liver-specific. The two other mammalian isozymes PKM1 and PKM2 are produced from the *PKM* gene ("M gene") by alternative splicing events controlled by hnRNP proteins. The PKM2 isozyme is expressed in fetal tissues and in adult proliferating cells such as cancer cells. Both PKR and PKM2 are in fact expressed in proerythroblasts. However, upon erythroid differentiation and maturation, PKM2 gradually is decreased in expression and progressively replaced by PKR in mature erythrocytes.

Clinically, hereditary PKR deficiency disorder manifests as non-spherocytic hemolytic anemia. The clinical severity of this disorder ranges from no observable symptoms in fully-compensated hemolysis to potentially fatal severe anemia requiring chronic transfusions and/or splenectomy at early development or during physiological stress or serious infections. Most affected individuals, who are asymptomatic, paradoxically due to enhanced oxygen-transfer capacity, do not require any treatment. However, for some of the most severe cases, while extremely rare population-wise with estimated prevalence of 51 per million (Beutler, E. Blood 2000, 95 (11), 3585-8), there is no disease-modifying treatment available for these patients other than palliative care (Tavazzi, D. et al., Pediatr Ann 2008, 37 (5), 303-10). These hereditary non-spherocytic hemolytic anemia (HNSHA) patients present a clear unmet medical need.

Heterogenous genetic mutations in PKR lead to dysregulation of its catalytic activity. Since the initial cloning of PKR and report of a single point mutation Thr³⁸⁴>Met associated with a HNSHA patient (Kanno, H. et al., Proc Natl Acad Sci U S A 1991, 88 (18), 8218-21), there are now nearly 200 different reported mutations associated with this disease reported worldwide (Zanella, A. et al., Br J Haematol 2005, 130 (1), 11-25; Kedar, P., et al., Clin Genet 2009, 75 (2), 157-62; Fermo, E. et al., Br J Haematol 2005, 129 (6), 839-46; Pissard, S. et al., Br J Haematol 2006, 133 (6), 683-9). Although these mutations represent wide range genetic lesions that include deletional and transcriptional or translational abnormalities, by far the most common type is missense mutation in the coding region that one way or another affects conserved residues within domains that are structurally important for optimal catalytic function of PKR. The pattern of mutation prevalence seems to be unevenly distributed toward specific ethnic backgrounds. For instance, the most frequent codon substitutions reported for North American and European patients appear to be Arg⁴⁸⁶>Trp and Arg⁵¹⁰>Gln, while mutations Arg⁴⁷⁹>His, Arg⁴⁹⁰>Trp and Asp³³¹>Gly were more frequently found in Asian patients (Kedar, P., et al., Clin Genet 2009, 75 (2), 157-62).

Kung Charles et al. discloses that "AG-348 Activation of Pyruvate Kinase in Vivo enhances Red Cell Glycolysis in Mice" and Yang Hua et al. discloses "Phase I Single (SAD) and Multiple Ascending Dose (MAD) Studies of the Safety, Tolerability, Pharmacokinetics (PK) and Pyruvate Kinase-R, in Healthy Subjects" at the 56th Annual Meeting of the Am. Chem. Soc. Hematol.; San Francisco, CA, USA Dec. 06-09, 2014 ISSN: 0006-4971. Pyruvate Kinase Inhibitors for use in therapy are described in WO 2012/151451 A1.

### SUMMARY OF THE INVENTION

The present invention is directed to a pharmaceutical composition comprising Compound 1 or a pharmaceutically acceptable salt or hydrate thereof in an orally acceptable dosage form for use in a method for treating thalassemia in a subject in need thereof, comprising orally administering to the subject the pharmaceutical composition comprising Compound 1 or a pharmaceutically acceptable salt or hydrate thereof at a dose of about 60 mg to about 200 mg of Compound 1 once or twice daily to thereby treat thalassemia in the subject, wherein Compound 1 is N-(4-(4-(cyclopropylmethyl)piperazine-1-carbonyl)phenyl)quinoline-8-sulfonamide.

Furthermore, the present invention is directed to a pharmaceutical composition comprising Compound 1 or a pharmaceutically acceptable salt or hydrate thereof in an orally acceptable dosage form for use in a method for treating sickle cell anemia in a subject in need thereof, comprising orally administering to the subject the pharmaceutical composition comprising Compound 1 or a pharmaceutically acceptable salt or hydrate thereof at a dose of about 60 mg to about 200 mg of Compound 1 once or twice daily to thereby treat sickle cell anemia in the subject, wherein Compound 1 is N-(4-(4-(cyclopropylmethyl)piperazine-1-carbonyl)phenyl)quinoline-8-sulfonamide.

In one aspect, disclosed is a method of evaluating a subject, the method comprising: administering to the subject N-(4-(4-(cyclopropylmethyl)piperazine-1-carbonyl)phenyl)quinoline-8-sulfonamide (Compound 1); and acquiring a value for the level of Compound 1, the level of 2,3-diphosphoglycerate (2,3-DPG), the level of adenosine triphosphate (ATP), or the activity of PKR in the subject, to thereby evaluate the subject.

In some embodiments, the value for the level of Compound 1 is acquired by analyzing the plasma concentration of Compound 1.

In some embodiments, the level of 2,3-DPG is acquired by analyzing the blood concentration of 2,3-DPG.

In some embodiments, the level of ATP is acquired by analyzing the blood concentration of ATP.

In some embodiments, the activity of PKR is acquired by analyzing the blood concentration of a ¹³C-label in the blood. For example, ¹³C-labeled glucose is administered to a subject, and incorporated into certain glycolytic intermediates in the blood.

In some embodiments, the analysis is performed by sample analysis of bodily fluid, such as blood, by *e.g.,* mass spectroscopy, *e.g.* LC-MS.

In another aspect, disclosed is a method of evaluating a subject, the method comprising acquiring, e.g., directly acquiring, the value for the level of a compound N-(4-(4-(cyclopropylmethyl)piperazine-1-carbonyl)phenyl)quinoline-8-sulfonamide (Compound 1), the level of 2,3-DPG, the level of ATP, or the activity of PKR in a subject that has been treated with Compound 1, to thereby evaluate the subject. In some embodiments, acquiring comprises receiving a sample from the subject. In some embodiments, acquiring comprises transmitting the value to another party, *e.g.,* the party that administered Compound 1.

In some embodiments, the value for the level of Compound 1 is acquired by analyzing the plasma concentration of Compound 1.

In some embodiments, the level of 2,3-DPG is acquired by analyzing the blood concentration of 2,3-DPG.

In some embodiments, the level of ATP is acquired by analyzing the blood concentration of ATP.

In some embodiments, the activity of PKR is acquired by analyzing the blood concentration of ¹³C-label in the blood. For example, ¹³C-labeled glucose is administered to a subject, and incorporated into certain glycolytic intermediates in the blood.

In some embodiments, the analysis is performed by sample analysis of bodily fluid, such as blood, by *e.g.,* mass spectroscopy, *e.g.* LC-MS.

In some embodiments, the subject has been administered Compound 1 within a preselected period of less than 7 days, less than 6 days, less than 5 days, less than 4 days, less than 3 days, or less than 72 hours prior to the evaluation, e.g., less than 48 hours, less than 24 hours, less than 12 hours, less than 10 hours, less than 8 hours, less than 6 hours, less than 4 hours, less than 3 hours, less than 2 hours, less than 1.5 hours, less than 1 hour, less than 45 minutes, less than 30 minutes, or less than 15 minutes.

In some embodiments, the subject has been administered Compound 1, *e.g.,* orally, a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the subject has been administered Compound 1, *e.g.,* orally, a dose of about 50 mg to about 300 mg, *e.g.,* about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, the subject has been administered, e.g., orally, Compound 1 once or twice daily.

In some embodiments, the subject has been administered Compound 1, *e.g.,* orally, twice daily, e.g., about every 12 hours. In some embodiments, Compound 1 is administered to the subject at about 10 mg to about 1000 mg about every 12 hours, *e.g.,* about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, the subject has been administered Compound **1,** *e.g.,* orally, once daily, e.g., about every 24 hours. In some embodiments, Compound 1 is administered, e.g., orally, to the subject at about 60 mg to about 200 mg about every 24 hours, e.g., about 90 mg about every 24 hours, about 120 mg about every 24 hours, about 150 mg about every 24 hours, about 180 mg about every 24 hours, or about 200 mg about every 24 hours.

In some embodiments, the method comprises comparing the level of Compound 1, the level of 2,3-DPG, or the level of ATP to a reference standard.

In some embodiments, the activity of PKR is acquired by analyzing the blood concentration of ¹³C-label in the blood. For example, ¹³C-labeled glucose is administered to a subject, and incorporated into certain glycolytic intermediates in the blood.

In some embodiments, the value for the level of Compound 1 is acquired by analyzing the plasma concentration of Compound 1.

In some embodiments, Compound 1 is present in a detectable amount in the subject at least 2 hours, at least 3 hours, at least 4 hours, at least 5 hours, at least 6 hours, at least 7 hours, at least 8 hours, at least 9 hours, or at least 10 hours after administration to the subject.

In some embodiments, the level of 2,3-DPG is acquired by analyzing the blood concentration of 2,3-DPG.

In some embodiments, the level of ATP is acquired by analyzing the blood concentration of ATP.

In some embodiments, the activity of PKR is acquired by analyzing the blood concentration of a ¹³C-label in the blood. For example, ¹³C-labeled glucose is administered to a subject, and incorporated into certain glycolytic intermediates in the blood.

In some embodiments, the analysis is performed by sample analysis of bodily fluid, such as blood, by *e.g.,* mass spectroscopy, *e.g.* LC-MS.

In some embodiments, the reference standard for the level of Compound 1, the level of 2,3-DPG, the level of ATP, or the level of PRK activity is the level of Compound 1, the level of 2,3-DPG, the level of ATP, or the level of PRK activity prior to administration of Compound 1.

In some embodiments, the value for the level of Compound 1 is acquired by analyzing the plasma concentration of Compound 1.

In some embodiments, the level of 2,3-DPG is acquired by analyzing the blood concentration of 2,3-DPG.

In some embodiments, the level of ATP is acquired by analyzing the blood concentration of ATP.

In some embodiments, the activity of PKR is acquired by analyzing the blood concentration of a ¹³C-label in the blood. For example, ¹³C-labeled glucose is administered to a subject, and incorporated into certain glycolytic intermediates in the blood.

In some embodiments, the analysis is performed by sample analysis of bodily fluid, such as blood, by *e.g.,* mass spectroscopy, *e.g.* LC-MS.

In some embodiments, the plasma concentration of Compound 1 is from about 10,000 ng/mL to about 1 ng/mL, e.g., about 1000 ng/mL to about 10 ng/mL.

In some embodiments, the blood concentration of 2,3-DPG is reduced by at least about 15% relative to the reference standard (e.g., from about 15% to about 60%). In some embodiments, the blood concentration of 2,3-DPG is reduced by at least about 15%, by at least about 20%, by at least about 25%, by at least about 30%, by at least about 35%, by at least about 40%, by at least about 45%, by at least about 50%, by at least about 55%, by at least about 60%.

In some embodiments, the blood concentration of 2,3-DPG is reduced for at least about 4 hours *(e.g.,* at least about 8 hours, at least about 12 hours, at least about 16 hours, at least about 20 hours, at least about 24 hours, at least about 36 hours, at least about 48 hours, at least about 72 hours or longer).

In some embodiments, the blood concentration of 2,3-DPG is reduced by at least about 15% relative to the reference standard *(e.g.,* from about 15% to about 60%). In some embodiments, the blood concentration of 2,3-DPG is reduced by at least about 15%, by at least about 20%, by at least about 25%, by at least about 30%, by at least about 35%, by at least about 40%, by at least about 45%, by at least about 50%, by at least about 55%, by at least about 60%, for at least about 4 hours *(e.g.,* at least about 8 hours, at least about 12 hours, at least about 16 hours, at least about 20 hours, at least about 24 hours, at least about 36 hours, at least about 48 hours, at least about 72 hours or longer).

In some embodiments, the method comprises administering an amount of Compound 1 sufficient to provide a blood concentration of 2,3-DPG that is reduced by at least 15% relative to the reference standard (e.g., from about 15% to about 60%). In some embodiments, the blood concentration of 2,3-DPG is reduced by at least about 15%, by at least about 20%, by at least about 25%, by at least about 30%, by at least about 35%, by at least about 40%, by at least about 45%, by at least about 50%, by at least about 55%, by at least about 60%.

In some embodiments, a single administration of Compound 1 is sufficient to provide a blood concentration of 2,3-DPG reduced by at least 15% relative to the reference standard (e.g., from about 15% to about 60%). In some embodiments, the blood concentration of 2,3-DPG is reduced by at least about 15%, by at least about 20%, by at least about 25%, by at least about 30%, by at least about 35%, by at least about 40%, by at least about 45%, by at least about 50%, by at least about 55%, by at least about 60%.
In another aspect, disclosed is Compound 1 for use in a method of treating a subject for a disorder, e.g., hereditary non-spherocytic hemolytic anemia; sickle cell anemia; thalassemia, e.g. beta-thalassemia; hereditary spherocytosis; hereditary elliptocytosis; sbetalipoproteinemia; Bassen-Kornzweig syndrome; or paroxysmal nocturnal hemoglobinuria, comprising administering to the subject an amount of Compound 1 sufficient to provide a blood concentration of 2,3-DPG reduced by at least 15% relative to the reference standard (e.g., from about 15% to about 60%). In some embodiments, the blood concentration of 2,3-DPG is reduced by at least about 15%, by at least about 20%, by at least about 25%, by at least about 30%, by at least about 35%, by at least about 40%, by at least about 45%, by at least about 50%, by at least about 55%, by at least about 60%.

In some embodiments, the reference standard is, *e.g.,* the 2,3-DPG level or the blood ATP level, in a diseased human, *e.g.,* a human having a metabolic disorder or a blood disorder, *e.g.,* a human diagnosed with pyruvate kinase deficiency (PKD). In some embodiments, the reference standard is, *e.g.,* a baseline level, *e.g.,* the 2,3-DPG level or the blood ATP level, in the subject prior to administration with Compound 1.

In some embodiments, the blood concentration of 2,3-DPG is reduced for at least about 4 hours *(e.g.,* at least about 8 hours, at least about 12 hours, at least about 16 hours, at least about 20 hours, at least about 24 hours, at least about 36 hours, at least about 48 hours, at least about 72 hours or longer).

In some embodiments, the subject has been administered Compound 1 within a preselected period of less than 7 days, less than 6 days, less than 5 days, less than 4 days, less than 3 days, or less than 72 hours prior to the evaluation, e.g., less than 48 hours, less than 24 hours, less than 12 hours, less than 10 hours, less than 8 hours, less than 6 hours, less than 4 hours, less than 3 hours, less than 2 hours, less than 1.5 hours, less than 1 hour, less than 45 minutes, less than 30 minutes, or less than 15 minutes.

In some embodiments, the subject is evaluated less than 72 hours, less than 48 hours, less than 24 hours, less than 12 hours, less than 10 hours, less than 8 hours, less than 6 hours, less than 4 hours, less than 3 hours, less than 2 hours, less than 1.5 hours, less than 1 hour, less than 45 minutes, less than 30 minutes, or less than 15 minutes, after administration of Compound 1.

In some embodiments, a single administration of Compound 1 is sufficient to provide a blood concentration of 2,3-DPG reduced by at least 15% relative to the reference standard (e.g., from about 15% to about 60%). In some embodiments, the blood concentration of 2,3-DPG is reduced by at least about 15%, by at least about 20%, by at least about 25%, by at least about 30%, by at least about 35%, by at least about 40%, by at least about 45%, by at least about 50%, by at least about 55%, by at least about 60%. In an embodiment, the blood concentration of 2,3-DPG is reduced for at least about 4 hours *(e.g.,* at least about 8 hours, at least about 12 hours, at least about 16 hours, at least about 20 hours, at least about 24 hours, at least about 36 hours, at least about 48 hours, at least about 72 hours or longer).

In some embodiments, the subject has been administered Compound 1, *e.g.,* orally, a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the subject has been administered Compound 1, *e.g.,* orally, a dose of about 50 mg to about 300 mg, *e.g.,* about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, the subject has been administered, e.g., orally, Compound 1 once or twice daily.

In some embodiments, the subject has been administered Compound 1, *e.g.,* orally, twice daily, e.g., about every 12 hours. In some embodiments, Compound 1 is administered to the subject at about 10 mg to about 1000 mg about every 12 hours, *e.g.,* about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, the subject has been administered Compound 1, *e.g.,* orally, once daily, e.g., about every 24 hours. In some embodiments, Compound 1 is administered, e.g., orally, to the subject at about 60 mg to about 200 mg about every 24 hours, e.g., about 90 mg about every 24 hours, about 120 mg about every 24 hours, about 150 mg about every 24 hours, about 180 mg about every 24 hours, or about 200 mg about every 24 hours.
In another aspect, disclosed is Compound 1 for use in a method of treating a subject for a disorder, e.g., hereditary non-spherocytic hemolytic anemia; sickle cell anemia; thalassemia, e.g. beta-thalassemia; hereditary spherocytosis; hereditary elliptocytosis; abetalipoproteinemia; Bassen-Kornzweig syndrome; or paroxysmal nocturnal hemoglobinuria, the method comprising orally administering to the subject a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, e.g., about 30 mg, about 120 mg, about 360 mg, about 700 mg, about 1400 mg, about 2500 mg, of Compound 1.

In some embodiments, the disorder is hereditary non-spherocytic hemolytic anemia.

In some embodiments, the disorder is sickle cell anemia.

In some embodiments, the disorder is thalassemia, e.g., beta-thalassemia.

In some embodiments, the disorder is hereditary spherocytosis.

In some embodiments, the disorder is hereditary elliptocytosis.

In some embodiments, the disorder is abetalipoproteinemia.

In some embodiments, the disorder is Bassen-Kornzweig syndrome.

In some embodiments, the disorder is paroxysmal nocturnal hemoglobinuria.

In some embodiments, the method comprises administering, e.g., orally, to the subject a dose of about 50 mg to about 300 mg, e.g., about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, Compound 1 is administered once or twice daily.

In some embodiments, Compound 1 is administered, e.g., orally, twice daily, e.g., about every 12 hours. In some embodiments, Compound 1 is administered to the subject at about 10 mg to about 1000 mg about every 12 hours, *e.g.,* about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, Compound 1 is administered, e.g., orally, once daily, *e.g.,* about every 24 hours. In some embodiments, Compound 1 is administered, *e.g.,* orally, to the subject at about 60 mg to about 200 mg about every 24 hours, e.g., about 90 mg about every 24 hours, about 120 mg about every 24 hours, about 150 mg about every 24 hours, about 180 mg about every 24 hours, or about 200 mg about every 24 hours.

Treatment methods described herein can additionally comprise various evaluation steps prior to and/or following treatment with Compound 1.

In some embodiments, prior to and/or after treatment with Compound 1, the method further comprises the step of evaluating PK and PD parameters (e.g., plasma concentration of Compound 1, 2,3-DPG and/or ATP). This evaluation may be achieved by sample analysis of bodily fluid, such as blood by *e.g.,* mass spectroscopy, *e.g.* LC-MS.

In another aspect, disclosed is an oral dosage unit of Compound 1, wherein the oral dosage unit consists of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the oral dosage unit consists of about 50 mg to about 300 mg, e.g., about 50 mg, about 75 mg, about 100 mg, about 125 mg, about 150 mg, about 175 mg, 200 mg, about 225 mg, about 250 mg, about 275 mg, about 300 mg, of Compound 1.

In another aspect, disclosed is a method of evaluating a subject, the method comprising administering to the subject N-(4-(4-(cyclopropylmethyl)piperazine-1-carbonyl)phenyl)quinoline-8-sulfonamide (Compound 1) and acquiring information regarding the occurance of an adverse event (AE) to thereby evaluate the subject.

In an embodiment, the adverse event is selected from headache, nausea, vomiting, and upper respiratory tract infection. In an embodiment, the adverse event is nausea. In an embodiment, the adverse event is vomiting. In an embodiment, the adverse event is upper respiratory tract infection.

In some embodiments, the subject has been administered Compound **1,** *e.g.,* orally, a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the subject has been administered Compound 1, *e.g.,* orally, a dose of about 50 mg to about 300 mg, *e.g.,* about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, the subject has been administered, e.g., orally, Compound 1 once or twice daily.

In some embodiments, the subject has been administered Compound 1, *e.g.,* orally, twice daily, e.g., about every 12 hours. In some embodiments, Compound 1 is administered to the subject at about 10 mg to about 1000 mg about every 12 hours, *e.g.,* about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, the subject has been administered Compound 1, *e.g.,* orally, once daily, e.g., about every 24 hours. In some embodiments, Compound 1 is administered, e.g., orally, to the subject at about 60 mg to about 200 mg about every 24 hours, e.g., about 90 mg about every 24 hours, about 120 mg about every 24 hours, about 150 mg about every 24 hours, about 180 mg about every 24 hours, or about 200 mg about every 24 hours.
Further disclosed is (1) Compound 1 or a pharmaceutically acceptable salt thereof; (2) a composition comprising Compound I or a salt thereof and a carrier; or (3) a pharmaceutical composition comprising Compound 1 or a pharmacetically acceptable salt thereof and a pharmaceutically acceptable carrier for use in a method for increasing the lifetime of red blood cells (RBCs) in need thereof comprising contacting blood with an effective amount of (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier.

In some embodiments, the method comprises orally administering to the subject a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the method comprises administering, e.g., orally, to the subject a dose of about 50 mg to about 300 mg, *e.g.,* about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, Compound 1 is administered once or twice daily.

In some embodiments, Compound 1 is administered, e.g., orally, twice daily, *e.g.,* about every 12 hours. In some embodiments, Compound 1 is administered to the subject at about 10 mg to about 1000 mg about every 12 hours, e.g., about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, Compound 1 is administered, e.g., orally, once daily, *e.g.,* about every 24 hours. In some embodiments, Compound 1 is administered, *e.g.,* orally, to the subject at about 60 mg to about 200 mg about every 24 hours, e.g., about 90 mg about every 24 hours, about 120 mg about every 24 hours, about 150 mg about every 24 hours, about 180 mg about every 24 hours, or about 200 mg about every 24 hours.

Further disclosed is (1) Compound 1 or a pharmaceutically acceptable salt thereof; (2) a composition comprising Compound I or a salt thereof and a carrier; or (3) a pharmaceutical composition comprising Compound 1 or a pharmacetically acceptable salt thereof and a pharmaceutically acceptable carrier for use in a method for regulating 2,3-diphosphoglycerate levels, e.g., reducing 2,3-diphosphoglycerate levels, in blood in need thereof comprising contacting blood with an effective amount of (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier.

In some embodiments, the method comprises orally administering to the subject a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the method comprises administering, e.g., orally, to the subject a dose of about 50 mg to about 300 mg, *e.g.,* about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, Compound 1 is administered once or twice daily.

In some embodiments, Compound 1 is administered, e.g., orally, twice daily, *e.g.,* about every 12 hours. In some embodiments, Compound 1 is administered to the subject at about 10 mg to about 1000 mg about every 12 hours, e.g., about 10 mg to about 60 mg about every 12 hours, about 60 mg to about 200 mg about every 12 hours, about 200 mg to about 500 mg about every 12 hours, about 500 mg to about 1000 mg about every 12 hours, *e.g.,* about 15 mg about every 12 hours, about 60 mg about every 12 hours, about 120 mg about every 12 hours, about 360 mg about every 12 hours, about 700 mg about every 12 hours.

In some embodiments, Compound 1 is administered, e.g., orally, once daily, e.g., about every 24 hours. In some embodiments, Compound 1 is administered, e.g., orally, to the subject at about 60 mg to about 200 mg about every 24 hours, e.g., about 90 mg about every 24 hours, about 120 mg about every 24 hours, about 150 mg about every 24 hours, about 180 mg about every 24 hours, or about 200 mg about every 24 hours.

In another aspect, disclosed is (1) Compound 1 or a pharmaceutically acceptable salt thereof; (2) a composition comprising Compound I or a salt thereof and a carrier; or (3) a pharmaceutical composition comprising Compound 1 or a pharmacetically acceptable salt thereof and a pharmaceutically acceptable carrier for use in a method of treating a subject, the method comprising: administering to the subject a therapeutically effective amount of (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier; and acquiring a value for the level of Compound 1, the level of 2,3-diphosphoglycerate (2,3-DPG), the level of adenosine triphosphate (ATP), or the activity of PKR in the subject, to thereby treat the subject.

In some embodiments, the method comprises orally administering to the subject a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the method comprises administering, e.g., orally, to the subject a dose of about 50 mg to about 300 mg, e.g., about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, Compound 1 is administered once or twice daily.

In some embodiments, Compound 1 is administered, e.g., orally, twice daily, e.g., about every 12 hours. In some embodiments, Compound 1 is administered to the subject at about 10 mg to about 1000 mg about every 12 hours, e.g., about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, Compound 1 is administered, e.g., orally, once daily, *e.g.,* about every 24 hours. In some embodiments, Compound 1 is administered, *e.g.,* orally, to the subject at about 60 mg to about 200 mg about every 24 hours, e.g., about 90 mg about every 24 hours, about 120 mg about every 24 hours, about 150 mg about every 24 hours, about 180 mg about every 24 hours, or about 200 mg about every 24 hours.

In some embodiments, the value for the level of Compound 1 is acquired by analyzing the plasma concentration of Compound 1.

In some embodiments, the level of 2,3-DPG is acquired by analyzing the blood concentration of 2,3-DPG.

In some embodiments, the level of ATP is acquired by analyzing the blood concentration of ATP.

In some embodiments, the activity of PKR is acquired by analyzing the blood concentration of a ¹³C-label in the blood. For example, ¹³C-labeled glucose is administered to a subject, and incorporated into certain glycolytic intermediates in the blood.

In some embodiments, the analysis is performed by sample analysis of bodily fluid, such as blood, by *e.g.,* mass spectroscopy, *e.g.* LC-MS.

In another aspect, disclosed is (1) Compound 1 or a pharmaceutically acceptable salt thereof; (2) a composition comprising Compound I or a salt thereof and a carrier; or (3) a pharmaceutical composition comprising Compound 1 or a pharmacetically acceptable salt thereof and a pharmaceutically acceptable carrier for use in a method for treating pyruvate kinase deficiency (PKD) in a subject in need thereof, comprising administering to the subject a therapeutically effective amount of (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier, to thereby treat PKD in the subject.

In some embodiments, prior to, during, and/or after treatment with (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier, the method further comprises the step of evaluating for levels of Compound **1,** or for the levels of one or more intermediate(s) in the glycolysis pathway, e.g., evaluating for levels of one or more of 2,3-diphosphoglycerate (2,3-DPG), adenosine triphosphate (ATP), or another intermediate in the glycolysis pathway.

In some embodiments, the method comprises activating one or more isozymes of pyruvate kinase, *e.g.,* one or more of PKR, PKM2 and/or PKL isozymes.

In some embodiments, the method comprises activating wild type PKR isozyme and/or a mutant PKR isozyme.

In some embodiments, the method comprises orally administering to the subject a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the method comprises administering, e.g., orally, to the subject a dose of about 50 mg to about 300 mg, *e.g.,* about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, Compound 1 is administered once or twice daily.

In some embodiments, Compound 1 is administered, e.g., orally, twice daily, *e.g.,* about every 12 hours. In some embodiments, Compound 1 is administered to the subject at about 10 mg to about 1000 mg about every 12 hours, e.g., about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, Compound 1 is administered, e.g., orally, once daily, *e.g.,* about every 24 hours. In some embodiments, Compound 1 is administered, *e.g.,* orally, to the subject at about 60 mg to about 200 mg about every 24 hours, e.g., about 90 mg about every 24 hours, about 120 mg about every 24 hours, about 150 mg about every 24 hours, about 180 mg about every 24 hours, or about 200 mg about every 24 hours.

In another aspect, disclosed is (1) Compound 1 or a pharmaceutically acceptable salt thereof; (2) a composition comprising Compound I or a salt thereof and a carrier; or (3) a pharmaceutical composition comprising Compound 1 or a pharmacetically acceptable salt thereof and a pharmaceutically acceptable carrier for use in a method of activating pyruvate kinase in a subject in need thereof, comprising administering to the subject a
therapeutically effective amount of (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier, to thereby activate pyruvate kinase in the subject.

In some embodiments, the method comprises activating one or more isozymes of pyruvate kinase, *e.g.,* one or more of PKR, PKM2 and/or PKL isozymes.

In some embodiments, the method comprises activating wild type PKR isozyme and/or a mutant PKR isozyme. In some embodiments, the mutant PKR isozyme is selected from G332S, G364D, T384M, G37E, R479H, R479K, R486W, R532W, R510Q, I90N, and R490W.

In some embodiments, the method comprises orally administering to the subject a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the method comprises administering, e.g., orally, to the subject a dose of about 50 mg to about 300 mg, e.g., about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, Compound 1 is administered once or twice daily.

In some embodiments, Compound 1 is administered, e.g., orally, twice daily, e.g., about every 12 hours. In some embodiments, Compound 1 is administered to the subject at about 10 mg to about 1000 mg about every 12 hours, *e.g.,* about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, Compound 1 is administered, e.g., orally, once daily, *e.g.,* about every 24 hours. In some embodiments, Compound 1 is administered, *e.g.,* orally, to the subject at about 60 mg to about 200 mg about every 24 hours, e.g., about 90 mg about every 24 hours, about 120 mg about every 24 hours, about 150 mg about every 24 hours, about 180 mg about every 24 hours, or about 200 mg about every 24 hours.

Further disclosed is (1) Compound 1 or a pharmaceutically acceptable salt thereof; (2) a composition comprising Compound I or a salt thereof and a carrier; or (3) a pharmaceutical composition comprising Compound 1 or a pharmacetically acceptable salt thereof and a pharmaceutically acceptable carrier for use in a method for treating hereditary non-spherocytic hemolytic anemia comprising administering to a subject in need thereof a therapeutically effective amount of (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier.

In some embodiments, prior to, during, and/or after treatment with (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier, the method further comprises the step of evaluating for the level of Compound 1, or for the levels of one or more intermediate(s) in the glycolysis pathway, e.g., evaluating for levels of one or more of 2,3-diphosphoglycerate (2,3-DPG), adenosine triphosphate (ATP), or another intermediate in the glycolysis pathway.

In some embodiments, the method comprises orally administering to the subject a dose of about 10 mg to about 3000 mg, e.g., about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the method comprises administering, e.g., orally, to the subject a dose of about 50 mg to about 300 mg, e.g., about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, Compound 1 is administered once or twice daily.

In some embodiments, Compound 1 is administered, e.g., orally, twice daily, *e.g.,* about every 12 hours. In some embodiments, Compound 1 is administered to the subject at about 10 mg to about 1000 mg about every 12 hours, e.g., about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, Compound 1 is administered, e.g., orally, once daily, *e.g.,* about every 24 hours. In some embodiments, Compound 1 is administered, *e.g.,* orally, to the subject at about 60 mg to about 200 mg about every 24 hours, e.g., about 90 mg about every 24 hours, about 120 mg about every 24 hours, about 150 mg about every 24 hours, about 180 mg about every 24 hours, or about 200 mg about every 24 hours.
Further disclosed is (1) Compound 1 or a pharmaceutically acceptable salt thereof; (2) a composition comprising Compound I or a salt thereof and a carrier; or (3) a pharmaceutical composition comprising Compound 1 or a pharmacetically acceptable salt thereof and a pharmaceutically acceptable carrier for use in a method for beating sickle cell anemia comprising administering to a subject in need thereof a therapeutically effective amount of (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier.

In some embodiments, prior to, during, and/or after treatment with (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier, the method further comprises the step of evaluating for the level of Compound 1, or for the levels of one or more intermediate(s) in the glycolysis pathway, e.g., evaluating for levels of one or more of 2,3-diphosphoglycerate (2,3-DPG), adenosine triphosphate (ATP), or another intermediate in the glycolysis pathway.

In some embodiments, the method comprises orally administering to the subject a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the method comprises administering, e.g., orally, to the subject a dose of about 50 mg to about 300 mg, *e.g.,* about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, Compound 1 is administered once or twice daily.

In some embodiments, Compound 1 is administered, e.g., orally, twice daily, *e.g.,* about every 12 hours. In some embodiments, Compound 1 is administered to the subject at about 10 mg to about 1000 mg about every 12 hours, e.g., about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, Compound 1 is administered, e.g., orally, once daily, e.g., about every 24 hours. In some embodiments, Compound 1 is administered, e.g., orally, to the subject at about 60 mg to about 200 mg about every 24 hours, e.g., about 90 mg about every 24 hours, about 120 mg about every 24 hours, about 150 mg about every 24 hours, about 180 mg about every 24 hours, or about 200 mg about every 24 hours.

Further disclosed is (1) Compound 1 or a pharmaceutically acceptable salt thereof; (2) a composition comprising Compound I or a salt thereof and a carrier; or (3) a pharmaceutical composition comprising Compound 1 or a pharmacetically acceptable salt thereof and a pharmaceutically acceptable carrier for use in a method for treating hemolytic anemia *(e.g.,* chronic hemolytic anemia caused by phosphoglycerate kinase deficiency, Blood Cells Mol Dis, 2011; 46(3):206) comprising administering to a subject in need thereof a therapeutically effective amount of (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier.

In some embodiments, prior to, during, and/or after treatment with (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier, the method further comprises the step of evaluating for the level of Compound 1, or for the levels of one or more intermediate(s) in the glycolysis pathway, e.g., evaluating for levels of one or more of 2,3-diphosphoglycerate (2,3-DPG), adenosine triphosphate (ATP), or another intermediate in the glycolysis pathway.

In some embodiments, the method comprises orally administering to the subject a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the method comprises administering, e.g., orally, to the subject a dose of about 50 mg to about 300 mg, *e.g.,* about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, Compound 1 is administered once or twice daily.

In some embodiments, Compound 1 is administered, e.g., orally, twice daily, e.g., about every 12 hours. In some embodiments, Compound 1 is administered to the subject at about 10 mg to about 1000 mg about every 12 hours, *e.g.,* about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, Compound 1 is administered, e.g., orally, once daily, *e.g.,* about every 24 hours. In some embodiments, Compound 1 is administered, *e.g.,* orally, to the subject at about 60 mg to about 200 mg about every 24 hours, e.g., about 90 mg about every 24 hours, about 120 mg about every 24 hours, about 150 mg about every 24 hours, about 180 mg about every 24 hours.

Further disclosed is (1) Compound 1 or a pharmaceutically acceptable salt thereof; (2) a composition comprising Compound I or a salt thereof and a carrier; or (3) a pharmaceutical composition comprising Compound 1 or a pharmacetically acceptable salt thereof and a pharmaceutically acceptable carrier for use in a method for treating thalassemia (e.g., beta-thalassemia), hereditary spherocytosis, hereditary elliptocytosis, abetalipoproteinemia (or Bassen-Kornzweig syndrome), paroxysmal nocturnal hemoglobinuria, acquired hemolytic anemia *(e.g.,* congenital anemias *(e.g.,* enzymopathies)), or anemia of chronic diseases comprising administering to a subject in need thereof a therapeutically effective amount of (1) N-(4-(4-(cyclopropylmethyl)piperazine-1-carbonyl)phenyl)quinoline-8-sulfonamide (Compound 1) or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier.

In some embodiments, prior to, during, and/or after treatment with (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier, the method further comprises the step of evaluating for the level of Compound 1, or for the levels of one or more intermediate(s) in the glycolysis pathway, e.g., evaluating for levels of one or more of 2,3-diphosphoglycerate (2,3-DPG), adenosine triphosphate (ATP), or another intermediate in the glycolysis pathway.

In some embodiments, the method comprises orally administering to the subject a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the method comprises administering, e.g., orally, to the subject a dose of about 50 mg to about 300 mg, *e.g.,* about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, Compound 1 is administered once or twice daily.

In some embodiments, Compound 1 is administered, e.g., orally, twice daily, *e.g.,* about every 12 hours. In some embodiments, Compound 1 is administered to the subject at about 10 mg to about 1000 mg about every 12 hours, e.g., about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, Compound 1 is administered, e.g., orally, once daily, *e.g.,* about every 24 hours. In some embodiments, Compound 1 is administered, *e.g.,* orally, to the subject at about 60 mg to about 200 mg about every 24 hours, e.g., about 90 mg about every 24 hours, about 120 mg about every 24 hours, about 150 mg about every 24 hours, about 180 mg about every 24 hours.

Further disclosed is (1) Compound 1 or a pharmaceutically acceptable salt thereof; (2) a composition comprising Compound I or a salt thereof and a carrier; or (3) a pharmaceutical composition comprising Compound 1 or a pharmacetically acceptable salt thereof and a pharmaceutically acceptable carrier for use in a method for treating diseases or conditions that are associated with increased 2,3-diphosphoglycerate levels *(e.g.,* liver diseases (Am J Gastroenterol, 1987;82(12):1283) and Parkinson's (J. Neurol, Neurosurg, and Psychiatry 1976,39:952) comprising administering to a subject in need thereof a therapeutically effective amount of (1) Compound 1 or a pharmaceutically acceptable salt thereof; (2) a composition comprising Compound 1 or a salt thereof and a carrier; or (3) a pharmaceutical composition comprising Compound 1 or a pharmaceutically acceptable salt thereof, and a pharmaceutically acceptable carrier.

In some embodiments, the method comprises orally administering to the subject a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg, of Compound 1.

In some embodiments, the method comprises administering, e.g., orally, to the subject a dose of about 50 mg to about 300 mg, *e.g.,* about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, Compound 1 is administered once or twice daily.

In some embodiments, Compound 1 is administered, e.g., orally, twice daily, *e.g.,* about every 12 hours. In some embodiments, Compound 1 is administered to the subject at about 10 mg to about 1000 mg about every 12 hours, e.g., about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, Compound 1 is administered, e.g., orally, once daily, *e.g.,* about every 24 hours. In some embodiments, Compound 1 is administered, *e.g.,* orally, to the subject at about 60 mg to about 200 mg about every 24 hours, e.g., about 90 mg about every 24 hours, about 120 mg about every 24 hours, about 150 mg about every 24 hours, about 180 mg about every 24 hours.

A compound N-(4-(4-(cyclopropylmethyl)piperazine-1-carbonyl)phenyl)quinoline-8-sulfonamide (Compound 1) and compositions comprising Compound 1 described herein are allosteric activators of PKR mutants and isoforms having lower activities compared to the wild type, thus are useful for methods of the present invention. Such mutations in PKR can affect enzyme activity (catalytic efficiency), regulatory properties (modulation by fructose bisphosphate (FBP)/ATP), and/or thermostability of the enzyme. Examples of such mutations are described in Valentini et al, JBC 2002. Some examples of the mutants that are activated by the compounds described herein include G332S, G364D, T384M, G37E, R479H, R479K, R486W, R532W, R510Q, I90N, and R490W. Without being bound by theory, Compound 1 affects the activities of PKR mutants by activating FBP non-responsive PKR mutants, restoring thermostability to mutants with decreased stability, or restoring catalytic efficiency to impaired mutants. Compound 1 is also an activator of wild type PKR.

In an embodiment, to increase the lifetime of the red blood cells, N-(4-(4-(cyclopropylmethyl)piperazine-1-carbonyl)phenyl)quinoline-8-sulfonamide (Compound 1), composition or pharmaceutical composition described herein is added directly to whole blood or packed cells extracorporeally or be provided to the subject *(e.g.,* the patient) directly *(e.g.,* by i.p., i.v., i.m., oral, inhalation (aerosolized delivery), transdermal, sublingual and other delivery routes). Without being bound by theory, Compound 1 increases the lifetime of the RBCs, thus counteract aging of stored blood, by impacting the rate of release of 2,3-DPG from the blood. A decrease in the level of 2,3-DPG concentration induces a leftward shift of the oxygen-hemoglobin dissociation curve and shifts the allosteric equilibribrium to the R, or oxygenated state, thus producing a therapeutic inhibition of the intracellular polymerization that underlies sickling by increasing oxygen affinity due to the 2,3-DPG depletion, thereby stabilizing the more soluble oxy-hemoglobin. Accordingly, in one embodiment, Compound 1 is useful as an antisickling agent. In another embodiment, to regulate 2,3-diphosphoglycerate, *e.g.* reduce 2,3-diphosphoglycerate levels, Compound 1 is added directly to whole blood or packed cells extracorporeally or be provided to the subject *(e.g.,* the patient) directly *(e.g.,* by i.p., i.v., i.m., oral, inhalation (aerosolized delivery), transdermal, sublingual and other delivery routes).

### BRIEF DESCRIPTION OF THE DRAWINGS

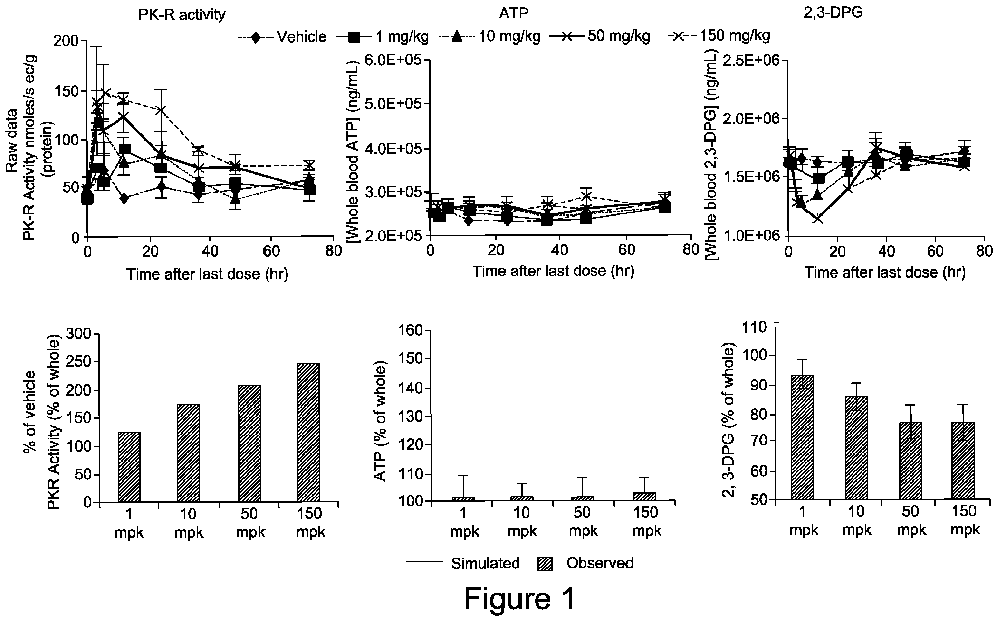

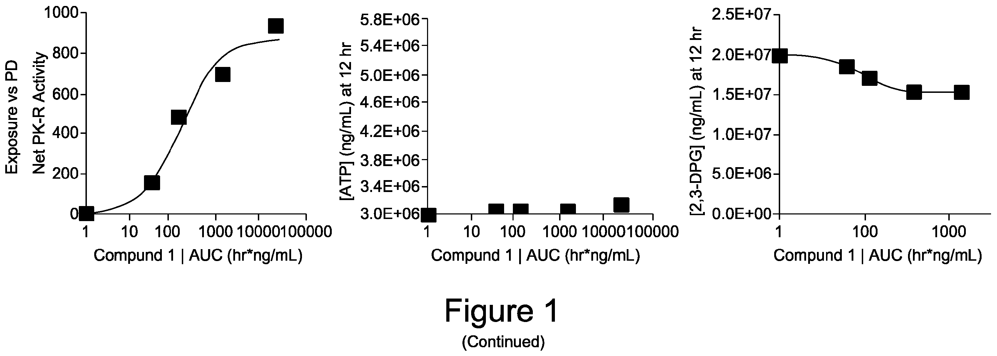

Figure 1 depicts line graphs showing PKR activity (left), ATP levels (center), and 2,3-DPG levels (right) in whole blood from C57/BL6 mice treated with a single dose of a compound N-(4-(4-(cyclopropylmethyl)piperazine-1-carbonyl)phenyl)quinoline-8-sulfonamide (Compound 1) at four dose levels (1 mpk, 10 mpk, 50 mpk, and 150 mpk). *Top row:* Raw data for PKR activity, ATP level, and 2,3-DPG level assesments; *Center row:* Percent changes of each marker for each dose normalized to vehicle treated; *Bottom row:* Pharmacokinetic / pharmacodynamic correlation between Compound 1 exposure in plasma and each marker.

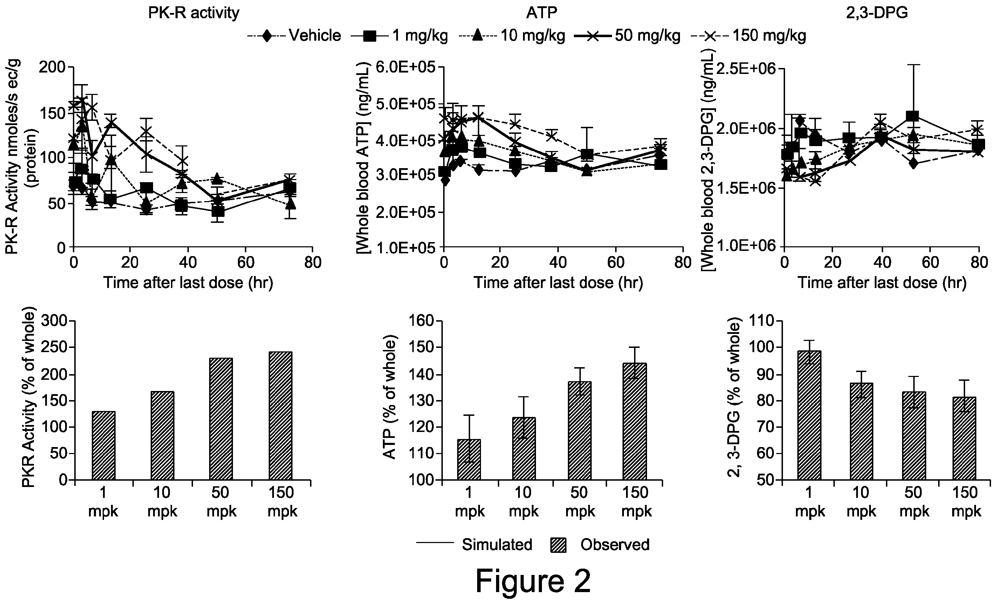

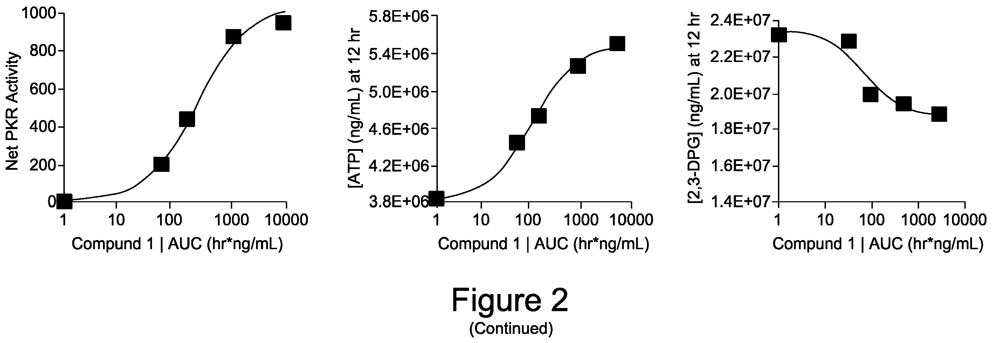

Figure 2 depicts line graphs showing PKR activity (left), ATP levels (center), and 2,3-DPG levels (right) in whole blood from C57/BL6 mice treated with a multiple doses (13 doses, BID) of a compound N-(4-(4-(cyclopropylmethyl)piperazine-1-carbonyl)phenyl)quinoline-8-sulfonamide (Compound 1) at four dose levels (1 mpk, 10 mpk, 50 mpk, and 150 mpk). *Top row:* Raw data for PKR activity, ATP level, and 2,3-DPG level assesments ; *Center row:* Percent changes of each marker for each dose normalized to vehicle treated; *Bottom row:* Pharmacokinetic / pharmacodynamic correlation between Compound 1 exposure in plasma and each marker.
Figure 3 depicts a schematic for the determination of PK flux activity in mice. C57/BL6 mice are administered 13 doses (BID) of a compound N-(4-(4-(cyclopropylmethyl)piperazine-1-carbonyl)phenyl)quinoline-8-sulfonamide (Compound 1, 100 mpk), and whole blood samples are removed over time. The blood samples are immediately incubated at 37 °C in the presence of [U-¹³C₆]-glucose, and the metabolites are extracted and quantified. The resulting data are subjected to a kinetic flux model to determine the overall change in carbon flow through the PKR reaction.
Figure 4 depicts a summary of the number of subjects experiencing adverse events (AEs) by treatment group in the SAD study, including the safety analysis set of both fed and fasted periods.
Figure 5 depicts a line graph showing the mean concentration-time profiles of a compound N-(4-(4-(cyclopropylmethyl)piperazine-1-carbonyl)phenyl)quinoline-8-sulfonamide (Compound 1) in human plasma following a single oral dose at 30 mg, 120 mg, 360 mg, 700 mg, 1400 mg, and 2500 mg.
Figure 6 depicts the pharmacokinetic (PK) parameter values of a compound N-(4-(4-(cyclopropylmethyl)piperazine-1-carbonyl)phenyl)quinoline-8-sulfonamide Compound 1 following a single oral dose (SAD study).
Figure 7 depicts a line graph showing the mean concentration-time profiles of 2,3-DPG in human blood following a single oral dose of placebo, 30 mg, 120 mg, 360 mg, 700 mg, 1400 mg, and 2500 mg of a compound N-(4-(4-(cyclopropylmethyl)piperazine-1-carbonyl)phenyl)quinoline-8-sulfonamide (Compound 1).
Figure 8 depicts a line graph showing the mean blood concentration-time profiles of 2,3-DPG following multiple oral doses of placebo, 120 mg, and 360 mg of a compound N-(4-(4-(cyclopropylmethyl)piperazine-1-carbonyl)phenyl)quinoline-8-sulfonamide (Compound 1) for cohorts 1 and 2 in the MAD study.
Figure 9 depicts a line graph showing the mean blood concentration-time profiles of ATP following multiple oral doses of placebo, 120 mg, and 360 mg of a compound N-(4-(4-(cyclopropylmethyl)piperazine-1-carbonyl)phenyl)quinoline-8-sulfonamide (Compound 1) for cohorts 1 and 2 in the MAD study.
Figure 10A depicts a line graph showing the change from baseline concentration-time profiles of 2,3-DPG following multiple oral doses of placebo, 15 mg (q12h), 60 mg (q12h), 120 mg (q12h), 360 mg (q12h), 700 mg (q12h) of a compound N-(4-(4-(cyclopropylmethyl)piperazine-1-carbonyl)phenyl)quinoline-8-sulfonamide (Compound 1), or a single dose of Compound 1 at 120 mg (q24h).
Figure 10B depicts a line graph showing the change from baseline concentration-time profiles of ATP following multiple oral doses of placebo, 15 mg (q12h), 60 mg (q12h), 120 mg (q12h), 360 mg (q12h), 700 mg (q12h) of a compound N-(4-(4-(cyclopropylmethyl)piperazine-1-carbonyl)phenyl)quinoline-8-sulfonamide (Compound 1), or a single dose of Compound 1 at 120 mg (q24h).
Figure 11 is a schematic illustrating the Phase 2 study described in the Examples herein. BID (q12h) = twice-daily (every 12 hours); DRT = data review team; PKR = pyruvate kinase red blood cell isoform; TBD = to be determined; w = weeks.
Figure 12 is a schematic outlining the pyruvate kinase R (PKR) enzymatic reaction and how several pharmacodynamic (PD) assessments contributes to a mechanistic understanding of the action of Compound 1.

### DETAILED DESCRIPTION OF THE INVENTION

The details of construction and the arrangement of components set forth in the following description or illustrated in the drawings are not meant to be limiting. Embodiments can be practiced or carried out in various ways. Also, the phraseology and terminology used herein is for the purpose of description and should not be regarded as limiting. The use of "including," "comprising," or "having," "containing", "involving", and variations thereof herein, is meant to encompass the items listed thereafter and equivalents thereof as well as additional items.

As used herein, the term "treat" means decrease, suppress, attenuate, diminish, arrest, or stabilize the development or progression of a disease/disorder *(e.g.e.g.,* hereditary non-spherocytic hemolytic anemia; sickle cell anemia; thalassemia, e.g. beta-thalassemia; hereditary spherocytosis; hereditary elliptocytosis; sbetalipoproteinemia; Bassen-Kornzweig syndrome; or paroxysmal nocturnal hemoglobinuria), lessen the severity of the disease/disorder (e.g., hereditary non-spherocytic hemolytic anemia; sickle cell anemia; thalassemia, e.g. beta-thalassemia; hereditary spherocytosis; hereditary elliptocytosis; sbetalipoproteinemia; Bassen-Kornzweig syndrome; or paroxysmal nocturnal hemoglobinuria) or improve the symptoms associated with the disease/disorder *(e.g., e.g.,* hereditary non-spherocytic hemolytic anemia; sickle cell anemia; thalassemia, e.g. beta-thalassemia; hereditary spherocytosis; hereditary elliptocytosis; sbetalipoproteinemia; Bassen-Kornzweig syndrome; or paroxysmal nocturnal hemoglobinuria).

As used herein, an amount of a compound N-(4-(4-(cyclopropylmethyl)piperazine-1-carbonyl)phenyl)quinoline-8-sulfonamide (Compound 1) effective to treat a disorder, or a "therapeutically effective amount" refers to an amount of the compound which is effective, upon single or multiple dose administration to a subject, in treating a cell, or in curing, alleviating, relieving or improving a subject with a disorder beyond that expected in the absence of such treatment.

As used herein, the dosing amount refers to the free base of Compound 1 or a pharmaceutically acceptable salt or solvate (e.g., hydrate) thereof.

As used herein, the term "subject" is intended to mean human. Exemplary human subjects include a human patient (referred to as a patient) having a disorder, *e.g.,* a disorder described herein or a normal subject.

As used herein, the term "acquire" or "acquiring" as the terms are used herein, refer to obtaining possession of a physical entity *(e.g.,* a sample, *e.g.,* blood sample or blood plasma sample), or a value, *e.g.,* a numerical value, by "directly acquiring" or "indirectly acquiring" the physical entity or value. "Directly acquiring" means performing a process *(e.g.,* an analytical method) to obtain the physical entity or value. "Indirectly acquiring" refers to receiving the physical entity or value from another party or source *(e.g.,* a third party laboratory that directly acquired the physical entity or value). Directly acquiring a value includes performing a process that includes a physical change in a sample or another substance, e.g., performing an analytical process which includes a physical change in a substance, e.g., a sample, performing an analytical method, *e.g.,* a method as described herein, *e.g.,* by sample analysis of bodily fluid, such as blood by, *e.g.,* mass spectroscopy, *e.g.* LC-MS.

### Methods of Treatment

Disclosed is Compound 1 for use in a method for treating or preventing a disease, condition or disorder as described herein (e.g., treating) comprising administering to a subject in need thereof N-(4-(4-(cyclopropylmethyl)piperazine-1-carbonyl)phenyl)quinoline-8-sulfonamide (Compound 1).

In some embodiments, the disorder is selected from hereditary non-spherocytic hemolytic anemia; sickle cell anemia; thalassemia, e.g. beta-thalassemia; hereditary spherocytosis; hereditary elliptocytosis; sbetalipoproteinemia; Bassen-Kornzweig syndrome; or paroxysmal nocturnal hemoglobinuria.

N-(4-(4-(cyclopropylmethyl)piperazine-1-carbonyl)phenyl)quinoline-8-sulfonamide (Compound 1) and compositions described herein can be administered to cells in culture, *e.g. in vitro* or *ex vivo,* or to a subject, *e.g., in vivo,* to treat, prevent, and/or diagnose a variety of disorders, including those described herein below.

### Compositions and Routes of Administration

The compositions delineated herein include the compound N-(4-(4-(cyclopropylmethyl)piperazine-1-carbonyl)phenyl)quinoline-8-sulfonamide (Compound 1), as well as additional therapeutic agents if present, in amounts effective for achieving a modulation of disease or disease symptoms, including those described herein.

The term "pharmaceutically acceptable carrier or adjuvant" refers to a carrier or adjuvant that may be administered to a patient, together with Compound 1, and which does not destroy the pharmacological activity thereof and is nontoxic when administered in doses sufficient to deliver a therapeutic amount of the compound.

Pharmaceutically acceptable carriers, adjuvants and vehicles that may be used in the pharmaceutical compositions provided herewith include, but are not limited to, ion exchangers, alumina, aluminum stearate, lecithin, self-emulsifying drug delivery systems (SEDDS) such as d-α-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as α-, β-, and γ-cyclodextrin, or chemically modified derivatives such as hydroxyalkylcyclodextrins, including 2- and 3-hydroxypropyl-β-cyclodextrins, or other solubilized derivatives may also be advantageously used to enhance delivery of compounds of the formulae described herein.

The pharmaceutical compositions provided herewith are orally administered in any orally acceptable dosage form including, but not limited to, capsules, tablets, emulsions and aqueous suspensions, dispersions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions and/or emulsions are administered orally, the active ingredient may be suspended or dissolved in an oily phase is combined with emulsifying and/or suspending agents. If desired, certain sweetening and/or flavoring and/or coloring agents may be added.

In some embodiments, Compound 1 is orally administering to the subject at a dose of about 10 mg to about 3000 mg, *e.g.,* about 10 mg to about 60 mg, about 60 mg to about 200 mg, about 200 mg to about 500 mg, about 500 mg to about 1200 mg, about 1200 mg to about 2000 mg, or about 2000 mg to about 3000 mg, *e.g.,* about 30 mg, about 120 mg, about 360 mg, about 700 mg, about 1400 mg, about 2500 mg.

In some embodiments, the method comprises administering, e.g., orally, to the subject a dose of about 50 mg to about 300 mg, *e.g.,* about 50 mg, about 75 mg, about 100 mg, about 125 mg, about 150 mg, about 175 mg, 200 mg, about 225 mg, about 250 mg, about 275 mg, about 300 mg, of Compound 1.

In some embodiments, Compound 1 is administered once or twice daily.

In some embodiments, Compound 1 is administered, e.g., orally, twice daily, *e.g.,* about every 12 hours. In some embodiments, Compound 1 is administered to the subject at about 10 mg to about 1000 mg about every 12 hours, e.g., about 10 mg to about 60 mg about every 12 hours, about 60 mg to about 200 mg about every 12 hours, about 200 mg to about 500 mg about every 12 hours, about 500 mg to about 1000 mg about every 12 hours, e.g., about 15 mg about every 12 hours, about 60 mg about every 12 hours, about 120 mg about every 12 hours, about 360 mg about every 12 hours, about 700 mg about every 12 hours.

In some embodiments, Compound 1 is administered, e.g., orally, once daily, *e.g.,* about every 24 hours. In some embodiments, Compound 1 is administered, *e.g.,* orally, to the subject at about 60 mg to about 200 mg about every 24 hours, e.g., about 90 mg about every 24 hours, about 120 mg about every 24 hours, about 150 mg about every 24 hours, about 180 mg about every 24 hours.

When the compositions provided herewith comprise a combination of Compound 1 and one or more additional therapeutic or prophylactic agents, both the compound and the additional agent should be present at dosage levels of between about 1 to 100%, and more preferably between about 5 to 95% of the dosage normally administered in a monotherapy regimen. The additional agents may be administered separately, as part of a multiple dose regimen, from the compounds provided herewith. Alternatively, those agents may be part of a single dosage form, mixed together with Compound 1 in a single composition.

Lower or higher doses than those recited above may be required. Specific dosage and treatment regimens for any particular patient will depend upon a variety of factors, including the activity of the specific compound employed, the age, body weight, general health status, sex, diet, time of administration, rate of excretion, drug combination, the severity and course of the disease, condition or symptoms, the patient's disposition to the disease, condition or symptoms, and the judgment of the treating physician.

Upon improvement of a patient's condition, a maintenance dose of a compound, composition or combination provided herewith may be administered, if necessary. Subsequently, the dosage or frequency of administration, or both, may be reduced, as a function of the symptoms, to a level at which the improved condition is retained when the symptoms have been alleviated to the desired level. Patients may, however, require intermittent treatment on a long-term basis upon any recurrence of disease symptoms.

### Patient Selection and Monitoring

The compound N-(4-(4-(cyclopropylmethyl)piperazine-1-carbonyl)phenyl)quinoline-8-sulfonamide (Compound 1) can activate wild type PKR and/or mutant PKRs. Some examples of the mutants that are activated by the compounds described herein include G332S, G364D, T384M, G37E, R479H, R479K, R486W, R532W, R510Q, I90N, and R490W. Accordingly, a patient and/or subject can be selected for treatment using Compound 1 by first evaluating the patient and/or subject to determine whether the subject carries a mutation in PKR (for examples, one of the mutations as described herein), and if the subject is determined to be carrying a mutation in PKR thus is in need of activation of the activity of the mutant PKR, then optionally administering to the subject Compound 1. A subject can be evaluated as carrying a mutation in PKR using methods known in the art. The subject can also be monitored, for example, subsequent to administration of Compound 1. In embodiments, the subject can be monitored for evaluation of certain PK/PD parameters of Compound 1 such as levels of Compound 1, levels of 2,3-DPG, or levels of ATP.

### EXAMPLES

### Example 1. Synthesis of N-(4-(4-(cyclopropylmethyl)piperazine-1-carbonyl)phenyl)quinoline-8-sulfonamide (Compound 1)

The synthesis of Compound 1 was carried out following the procedure described in U.S. Patent No. 8,785,450.

### Example 2. N-(4-(4-(cyclopropylmethyl)piperazine-1-carbonyl)phenyl)quinoline-8-sulfonamide (Compound 1) Activation of Pyruvate Kinase In Vivo Enhances Red Cell Glycolysis in Mice

Pyruvate kinase deficiency (PKD) is an autosomal recessive enzymopathy that is the most common cause of hereditary nonspherocytic hemolytic anemia (HNSHA). PKD is a rare disease characterized by a life-long chronic hemolysis with severe co-morbidities. It is hypothesized that insufficient energy production to maintain red cell membrane homeostasis promotes the chronic hemolysis. Treatment is generally palliative, focusing on the resultant anemia, and there are no approved drugs that directly target mutated pyruvate kinase.

Compound 1 is an allosteric activator of the red cell isoform of pyruvate kinase (PKR) that has recently entered Phase I clinical trials in normal healthy volunteers. Compound 1 increases the catalytic efficiency and enhances the protein stability of a spectrum of recombinantly expressed PKR mutant proteins that have been associated with PKD. PKD red cells are characterized by changes in metabolism associated with defective glycolysis, including a build-up of the upstream glycolytic intermediate 2,3-DPG and deficiency in the PKR product adenosine triphosphate (ATP). PKR flux, *e.g.* the rate of carbon flow through the PKR enzyme reaction, was examined in PKD patient or WT donor blood samples by incubation of whole blood with a stable isotope tracer, [U-¹³C₆]-glucose. At various time points after the addition of [U-¹³C₆]-glucose, metabolism was quenched and metabolites were extracted. Metabolite pool sizes and ¹³C label incorporation into glycolytic intermediates were monitored by LC/MS. The rate of label incorporation was found to be significantly slower in PKD patient red cells, consistent with decreased glycolytic activity. Treatment of PKD red cells with Compound 1 ex-vivo induces changes in metabolism consistent with increased glycolytic activity including reduced 2,3-DPG levels, increased ATP levels, and increased PKR enzyme activity levels.

The effect of Compound 1 on red cell metabolism in vivo was evaluated in mice. C57/BL6 mice were dosed by oral gavage either with a single dose, or with multiple doses (13 doses, BID) of Compound 1 for 7 days. Dose levels tested were 1 mpk, 10 mpk, 50 mpk, and 150 mpk. Following the last dose, mice were bled to evaluate drug exposure and pharmacodynamic markers including 2,3-DPG and ATP levels, and PKR activity. Compound 1 was demonstrated to be a well-behaved compound, with dose-proportional increase in exposure, both in the single dose and multiple dose studies. A single dose of Compound 1 resulted in a dose-dependent increase in PKR activity levels (Figure 1, left), concomitant with reduction in 2,3-DPG levels (Figure 1, right). There were no significant changes in ATP levels after a single administration of Compound 1 (Figure 1, center). In the multiple dose studies, similar changes in PKR activity (Figure 2, left) and 2,3-DPG levels were observed (Figure 2, right). In contrast to the single-dose study, ATP levels in the multiple dose study were observed to be robustly increased in a dose-dependent manner (Figure 2, center). The resulting pharmacokinetic/pharmacodynamic correlations between Compound 1 exposure in plasma and each pharmacodynamic marker (PKR activity as well as ATP and 2,3-DPG levels) for both the single dose and multiple dose studies further highlights these observations (Figures 1 and 2, lower panels).

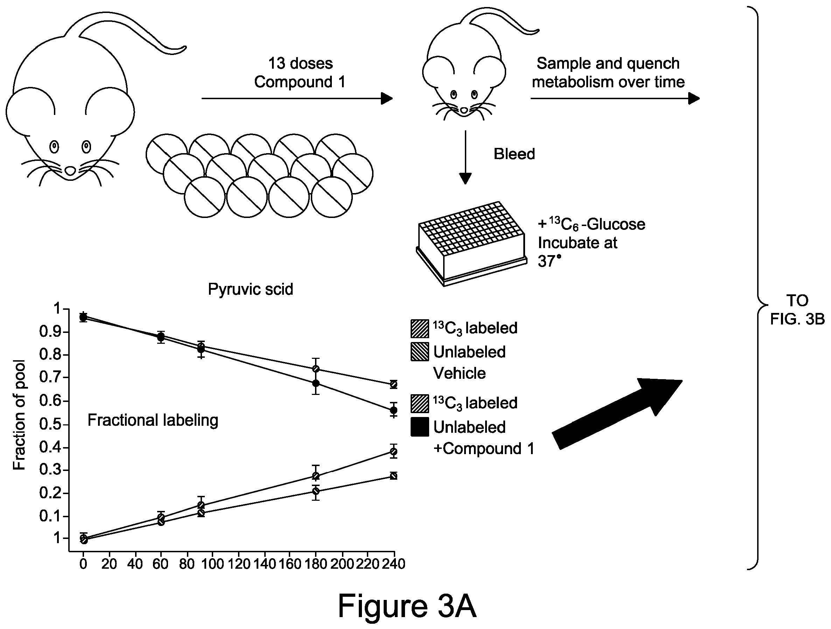

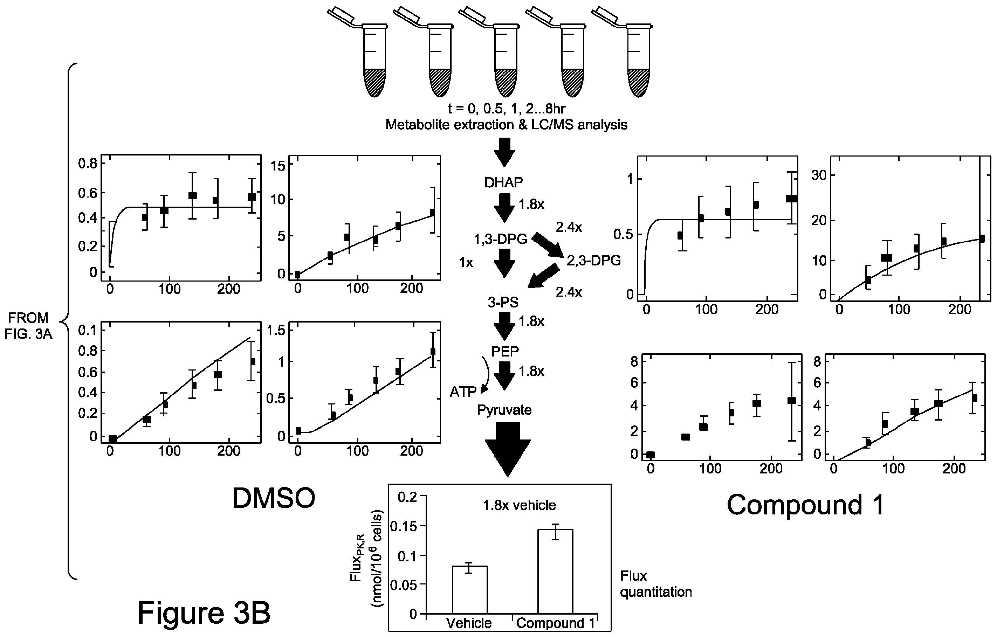

The effect of Compound 1 on PKR flux was assessed in whole blood from mice treated with Compound 1. C57BL/6 mice were dosed by oral gavage with Compound 1 at 100 mpk BID for 13 total doses. Whole blood was incubated with [U-¹³C₆]-glucose at 37 °C and the metabolite pool sizes and rate of ¹³C label incorporation into glycolytic intermediates were assessed. The data were subsequently analyzed using a mathematical kinetic flux model to quantify the overall change in carbon flow through the PKR reaction. Using this model, it was determined that Compound 1 treatment significantly increased glycolytic flux through the PKR reaction as depicted schematically in Figure 3.

Collectively, these data demonstrate that Compound 1 not only potently binds to and activates the PKR enzyme in vivo, but this enzyme activation induces enhanced glycolytic pathway activity in red cells that results in profound changes in cellular metabolism, as reflected in dramatically increased ATP levels and reduced 2,3-DPG levels. As Compound 1 has similar potency against the WT PKR enzyme as against tested mutant PKR enzymes in vitro, these data support the hypothesis that Compound 1 treatment may similarly enhance glycolytic activity in PKD patients and thus correct the underlying pathology of PKD.

### Example 3. Clinical Studies of the Safety, Tolerability, Pharmacokinetics (PK) and Pharmacodynamics (PD) of a Pyruvate Kinase-R Activator in Healthy Subjects

Compound 1 is a novel, first-in-class, small molecule allosteric activator of PK-R that directly targets the underlying metabolic defect in PKD. Pre-clinical studies demonstrated that Compound 1 increases the activity of both wild type and various mutated PK-R enzymes. The key objective of these first-in-human, Phase I, randomized, double-blind, placebo-controlled single and multiple ascending dose studies (SAD and MAD) are to identify a safe and pharmacodynamically active dose and schedule for Compound 1 to be used in subsequent clinical studies in subjects with pyruvate kinase deficiency.

### Methods

In the single ascending dose (SAD) study, healthy men and women (non-childbearing potential) aged 18-60 years were randomized to receive a single oral dose of Compound 1 or placebo (P). Key exclusion criteria included glucose 6-phosphate dehydrogenase deficiency, blood donation, blood loss of greater than 500 mL, or transfusion of blood or plasma within three months of screening. Six cohorts were evaluated, each containing 8 subjects (6 subjects receiving Compound 1, 2 subjects receiving placebo (P)), starting with 30 mg in cohort 1 followed by 120 mg, 360 mg, 700 mg, 1400 mg and 2500 mg in cohorts 2-6, respectively.

In the multiple ascending dose (MAD) study, 2 cohorts (120 mg BID and 360 mg BID) of 8 subjects each (6 subjects receiving Compound 1, 2 subjects receiving placebo (P)) have completed 14 days of dosing and 2 weeks of follow-up. In both studies, safety assessments included adverse events (AEs), vital signs, ECG and clinical laboratory parameters. Serial blood samples were drawn for assessment of PK and PD parameters (2,3-DPG and ATP) pre-dose and at regular intervals thereafter at multiple doses in both the SAD and MAD studies. Specifically, plasma concentrations of Compound 1 and blood concentrations of 2,3-DPG and ATP were analyzed by tandem mass spectrometry methods.

The MAD study was completed by assessing safety, tolerability, and pharmacokinetics/pharmacodynamics (PK/PD) of Compound 1 in healthy volunteers and to identify a dosing schedule for future trials in patients with PK deficiency. A phase 1, single-center, randomized, double-blind, placebo-controlled MAD study (ClinicalTrials.gov NCT02149966) was conducted in healthy men and women (18-60 years), in 6 sequential cohorts (each cohort: n=6 Compound 1, n=2 placebo). Subjects received twice daily oral doses of Compound 1 at 15 mg to 700 mg (q12h), or 120 mg once daily (q24hr) for 14 days with follow-up to Day 29. Adverse events (AEs), laboratory parameters, ECGs, and vital signs were monitored. Plasma concentrations of Compound 1 and whole blood 2,3-DPG and ATP levels were measured in serial blood samples for PK/PD assessment. Hormone levels were monitored due to pre-clinical data suggesting potential modulation.

### Results

### Single Ascending Dose (SAD) Study

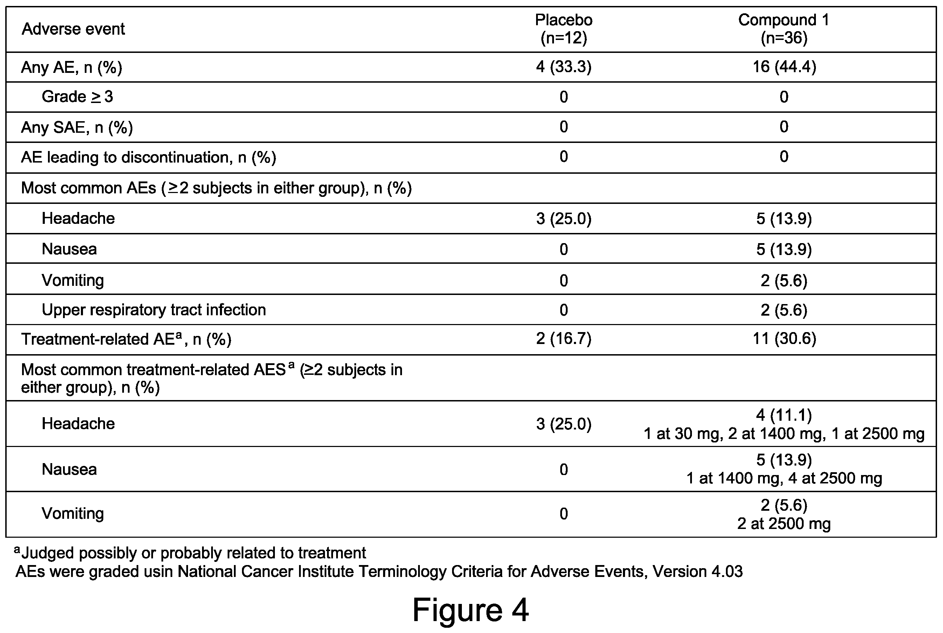

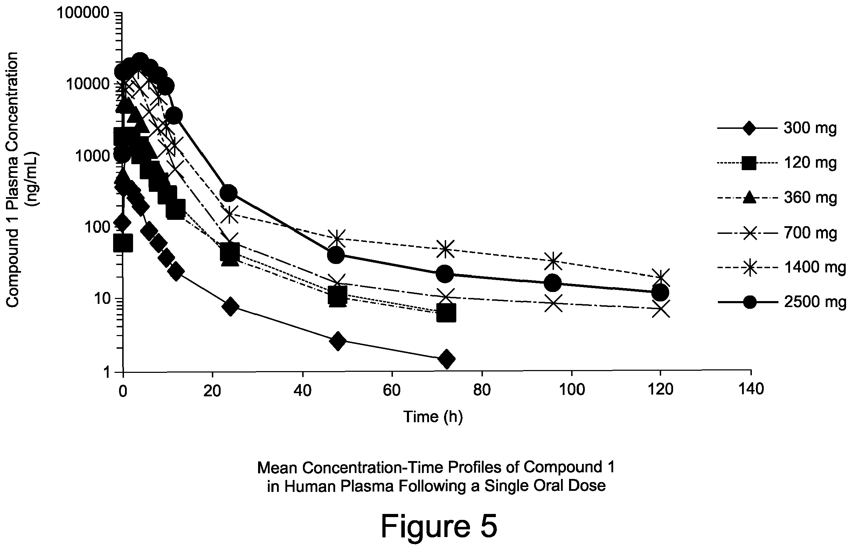

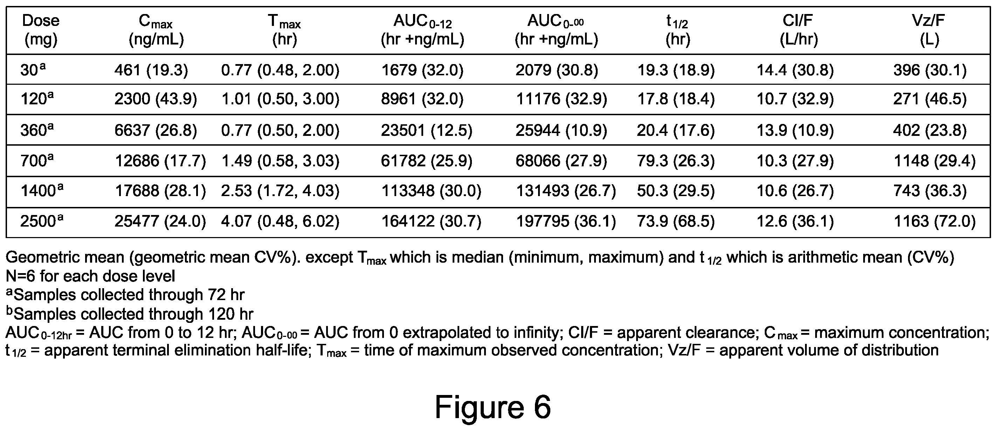

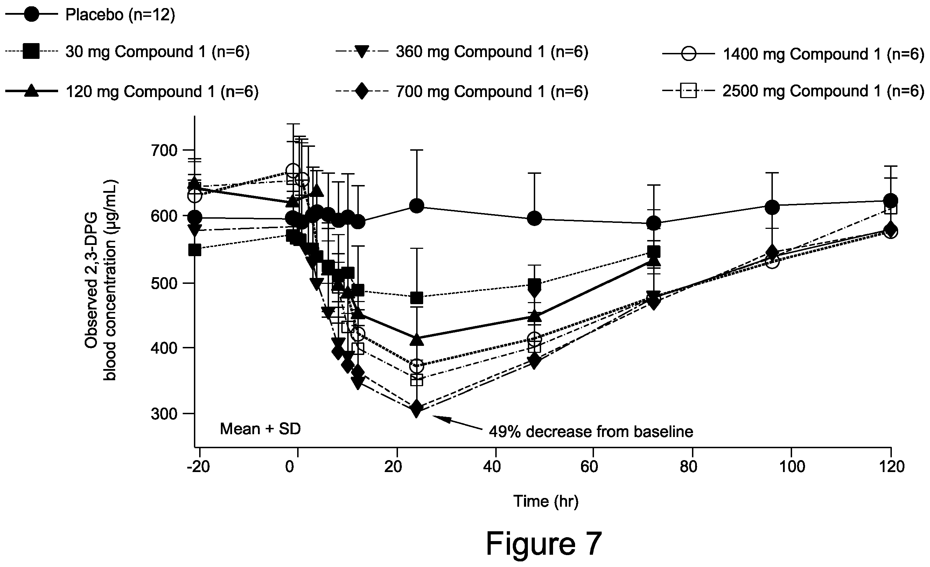

In the SAD, all 48 subjects enrolled completed the study, which included47 males and 1 female. These subjects represented a diverse racial and ethnic pool (15 White, 31 Black, 1 Asian, 1 Native Hawaiin or other Pacific Islander, wherein 7 subjects identify as Hispanic or Latino) and the mean age was about 40 years. Analysis of safety data indicated that 19/48 (39%) subjects receiving Compound 1 or placebo (P) under fasted and/or fed conditions experienced at least 1 treatment emergent adverse event (AE) during the study (Figure 4). All AEs were mild or moderate (Grade 1 and 2) in severity, and the most common were nausea (n=5; 10%) and headache (n=8; 17%). In the 2 completed MAD cohorts (13 males; 3 females; mean age 44 years) 8/16 (50%) of subjects receiving Compound 1 or placebo experienced 11 AEs. All AEs were mild (n=10) or moderate (n=1) and the most frequent were venipuncture bruises. There were no serious AEs, discontinuations due to AEs, or dose-limiting toxicities in either study. Maximum tolerated dose was not reached in the SAD and dose escalation continues in the MAD. In SAD cohorts 1-6, exposure to single doses of Compound 1 increased in a dose-proportional manner (mean plasma Cₘₐₓ, AUC₀₋₁₂ₕᵣ and AUC₀₋₇₂ₕᵣ) (Figure 5). Absorption was rapid, with a median Tₘₐₓ of 0.75-4.0 h. The pharmacokinetic parameter values of Compound 1 for each SAD cohort are summarized in Figure 6. As expected, Compound 1 had a rapid distribution or elimination phase during the first 12 hours following dosing, with an apparent half-life of approximately 2-4 hours (Figure 5). The mean apparent terminal half-life (t_{1/2}) ranged from 17.5-20.5 hours or 50-80 hours, when concentrations were measured for 72 or 120 hours, respectively (Figure 6). In addition, a dose-dependent decrease in the concentration of the pharmacodynamic marker 2,3-DPG was observed over 24 hours following exposure to Compound 1(with a 48% decrease), which returned to placebo levels after 72 hours (Figure 7). Preliminary results indicated that food has a minimal effect on the exposure to Compound 1.

### Multiple Ascending Dose (MAD) Study

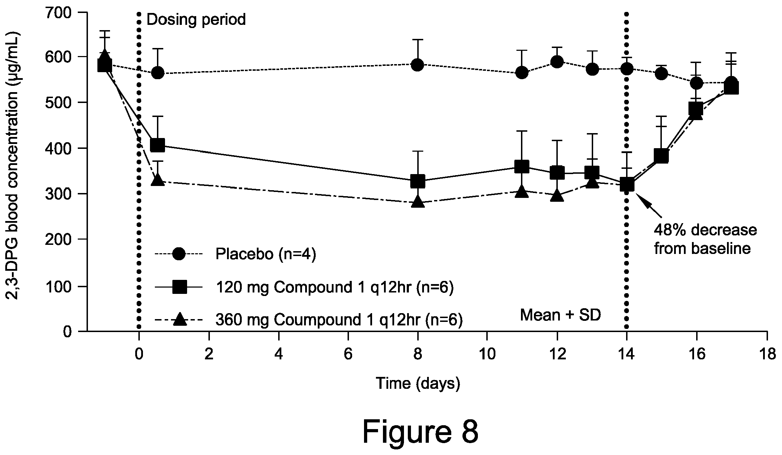

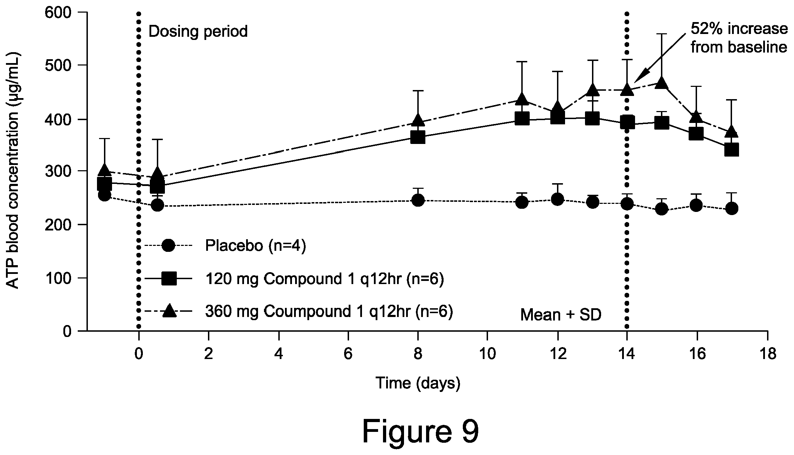

In the MAD study, the pharmacokinetic results for cohorts 1 and 2 on Day 1 were consistent with those of the SAD study. However, the pharmacokinetic parameter values of Compound 1 were lower on Day 14 compared with that on Day 1, suggesting that multiple doses of Compound 1 may result in an increase in the rate of drug metabolism. The decrease in exposure on Day 14 is consistent with pre-clinical data suggesting that Compound 1 is a moderate inducer of cytochrome P450 3A4 (CYP3A4), which is the major route of the oxidative metabolism of Compound 1. Similarly, decreases in 2,3-DPG levels were also observed after administration of the final dose in cohorts 1 and 2 of the MAD study. Concentrations of 2,3-DPG in the blood returned to placebo levels between 48 and 72 hours after the last dose (Figure 8). There were minimal increases in blood ATP levels after a single dose of Compound 1 in the SAD study. In contrast, there were substantial increases in ATP levels in the blood on Days 8-14 of subjects in cohorts 1 and 2 in the MAD study, and levels remained elevated through 72 hours after the last dose (Figure 9).

In the 6 sequential cohorts, 48 subjects (42 males and 6 females) with a mean age 41.5 (25-60) years were enrolled. Final, unblinded safety data showed ≥1 AE in 16 out of 36 (44%) subjects treated with Compound 1 and 4 out of the 12 (33%) placebo (P) subjects. Treatment related ≥1 AEs was noted in 11 out of the 36 (31%) subjects treated with Compound 1 and 3 out of the 12 (25%) placebo subjects. All treatment-related AEs were mild or moderate (only 1 grade 3 event) in severity and often reversible despite continued dosing. The most frequent Compound 1 related AEs were nausea and headache, 5 out of the 36 (14%) subjects for each (P: 0/12 (0%) nausea; headache 1 out of the 12 (8%)). Gastrointestinal AEs occurred in subjects treated with Compound 1 only at the highest dose, 700 mg q12h. One Grade 3 AE occurred (Compound 1 (700 mg q12h), elevated liver function tests (LFTs) which resolved after treatment discontinuation). There were four Compound 1 discontinuations: due to AEs in 2 subjects (Grade 2 drug eruption, 60 mg q12h; Grade 3 elevated LFTs, 700 mg q12h), and 2 subjects withdrew consent (both had Grade 1/2 nausea and Grade 1/1 vomiting, both at 700 mg q12h).

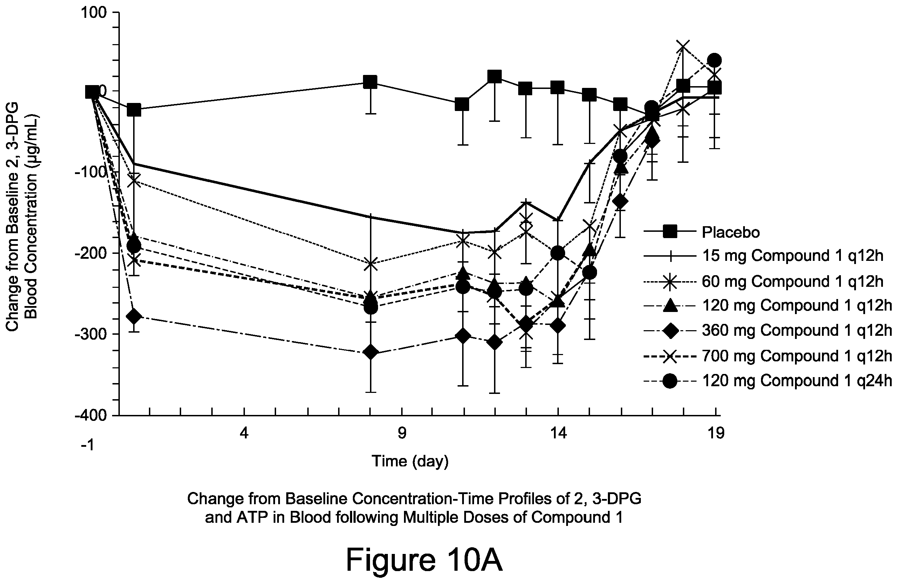

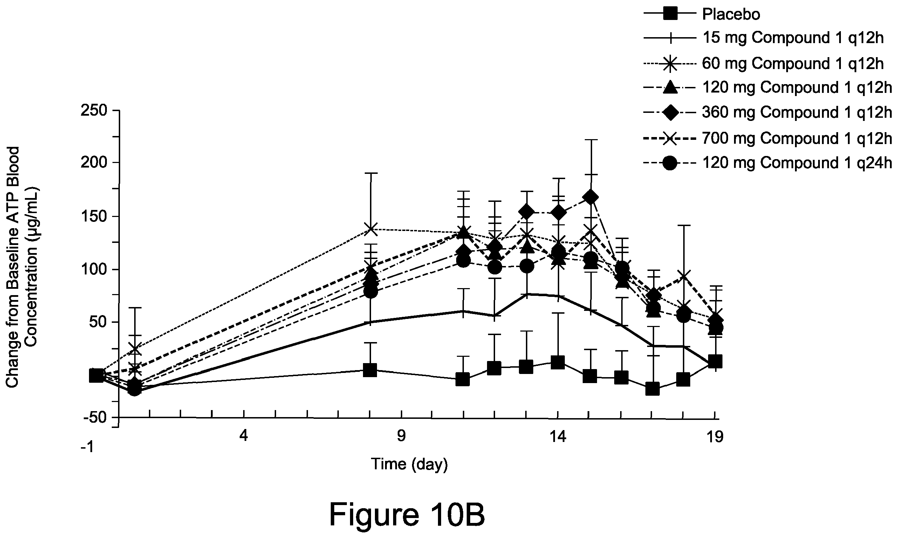

The highest well-tolerated dose was 360 mg q12h (doses between 360 and 700 were not explored). Compound 1 plasma exposure was dose dependent with low to moderate variability in the PK parameters of Compound 1 and its metabolite. There was a dose-dependent decrease in 2,3-DPG and increase in ATP with the effects plateauing at 360 mg q12h. Decrease in 2,3-DPG was robust after Dose 1, while the increase in ATP occurred gradually and was strongly evident at Day 8. Change from baseline in 2,3-DPG and ATP plateaued at ~300 µg/ml (~50% decrease) and ~175 µg/ml (~50% increase), respectively (Figures 10A and 10B, respectively). After the final Day 14 dose, 2,3-DPG returned to levels similar to baseline between 72 and 120 hours (Figure 10A). ATP levels remained elevated through 120 hours post-dose (Figure 10B).

Compound 1 had a favorable safety profile and was well-tolerated in healthy subjects based on preliminary analysis of subjects receiving a single dose up to 2500 mg or multiple BID doses up to 360 mg for up to 14 days. Compound 1 also demonstrated a desirable PK profile, with rapid absorption, low PK variability and dose-proportional exposure with PD effect as demonstrated on 2,3-DPG and ATP. There were no serious AEs, discontinuations due to AEs, or dose-limiting toxicities in the SAD study, and so far no serious AEs in the MAD study.

The dose-dependent changes in ATP and 2,3-DPG blood levels seen in these studies are consistent with increased activity of the glycolytic pathway, which represents the expected pharmacodynamic effect of Compound 1. These data are consistent with pre-clinical studies in mice described in Example 2. As Compound 1 has roughly equipotent biochemical activity against wild type and mutant PKR enzymes, the data support the hypothesis that Compound 1 may be able to enhance glycolytic activity in red blood cells of patients with PKD to address the underlying cause of the diseases.

As shown in Figure 10A there was a decrease in 2,3-DPG in blood with Compound 1. Mean 2,3-DPG blood levels generally decreased from baseline over the 12-hour post-dose period following the first dose of Compound 1 across the dose levels studied. The rate of decrease in 2,3-DPG levels was slower at lower doses. A large fraction of the decrease occurred after the first dose and the decrease reached its full extent within 7 days of dosing. Dose-related decreases in 2,3-DPG levels were observed with increasing doses of Compound 1 at low doses of 15 and 60 mg of Compound 1 q12h and reached a plateau over the 120 to 700 mg q12h dose levels, with minimal additional decreases with higher doses. The maximum decrease in 2,3-DPG levels was approximately 300 µg/mL, an approximately 50% decrease. The concentration of 2,3-DPG returned to baseline within 72 hours after the final dose of Compound 1.

As shown in Figure 10B there was an increase in ATP in blood with Compound 1. ATP levels increased during multiple dose administration of Compound 1. Any effect of Compound 1 on ATP levels during the 12 hours following the first dose was minimal. The increase in ATP levels reached its full extent within 10 days of dosing. Increases in ATP levels were observed with increasing AG-348 doses at low doses of 15 and 60 mg AG-348 q12h and reached a plateau over the 120 to 700 mg dose levels, with minimal additional increases with higher doses. The maximum increase in ATP levels was approximately 175 µg/mL, an approximately 50% increase. The concentration of ATP remained elevated for 120 hours after the final dose of Compound 1.

### Example 4. Clinical Studies of the Safety, Efficacy, Pharmacokinetics (PK), and Pharmacodynamics (PD) of a Pyruvate Kinase-R (PKR) Activator in Subjects with Pyruvate Kinase Deficiency

This example describes a Phase 2, open-label, two-arm, multicenter, randomized, dose-ranging study of Compound 1 in adult patients with pyruvate kinase deficiency (PK deficiency). This is the first study to be conducted in patients with PK deficiency. The key objective of this study is to evaluate the safety and tolerability of up to 24 weeks of Compound 1 administration in patients with PK deficiency.

### Methods

In this Phase 2, open label, two arm, multicenter, randomized, dose-ranging study, adult (male and female) patients with pyruvate kinase deficiency receive multiple doses of Compound 1 for up to 24 weeks. Pyruvate kinase deficiency in patients is confirmed by red blood cell (RBC) pyruvate kinase enzymatic assay. At Week 25, patients who safely tolerate Compound 1 and demonstrate clinical activity of Compound 1 may be eligible to roll over to a separate safety extension study for continued treatment. Patients who finish treatment at the end of 24 weeks or sooner will undergo follow-up assessment 4 weeks after the last dose of the study drug. Patients with toxicity suspected to be related to study drug will continue follow-up until the adverse event (AE) resolves, is declared chronic, or the patient is lost to follow-up.

### Patient Selection

Patients are screened prior to randomization and Day 1 of the treatment period to meet certain criteria. Patients included in the study are adults *(e.g.,* aged 18 or older) who have a medical history/diagnosis of pyruvate kinase deficiency and who are anemic but non-transfusion dependent.

### Randomization and Dosing

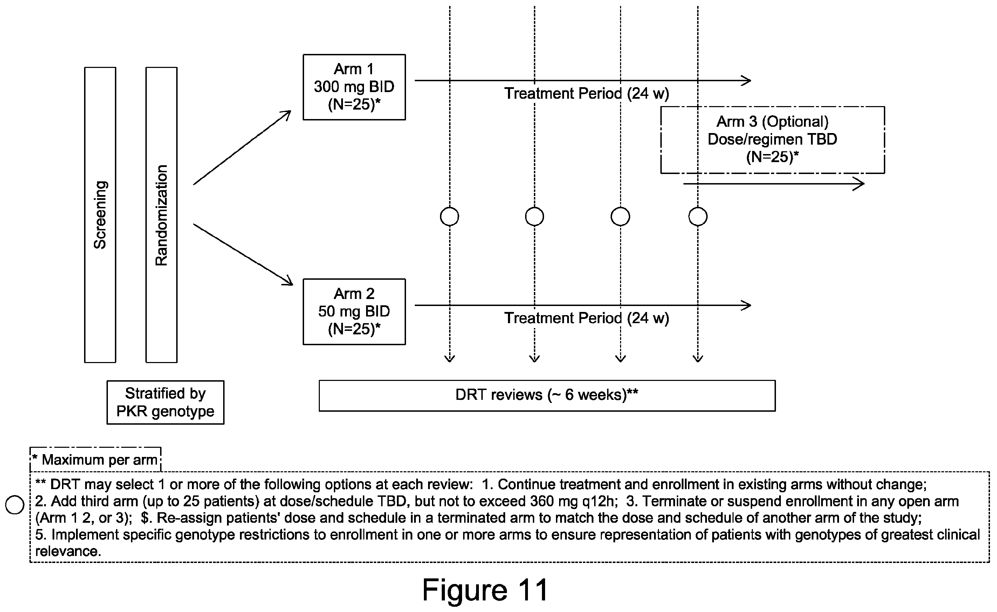

Initially, up to 25 patients are randomized on an open-label, 1:1 basis to each of two arms, *e.g.,* 25 patients per arm. In Arm 1, two twice-daily (BID) doses of Compound 1 are given-300 mg Compound 1 administered orally every 12 hours (q12h) (BID), *e.g.,* with a minimum of 10 hours between doses. In Arm 2, 50 mg of Compound 1 is administered orally q12h (BID). (See Figure 11) Starting with Day 1, dosing is continuous (e.g., there are no rest periods). Compound 1 is provided as a 25 mg or 100 mg (free-base equivalent) capsule of Compound 1. The number of capsules per dose will vary by assigned dose group. Patients will receive multiple oral (PO) doses of Compound 1 over a 24-week treatment period.

### Patient Assessments

Safety will be monitored on an on-going basis, *e.g.,* at regular intervals, or *ad hoc* as necessary. For example, adverse events (AEs), vital signs (VS), clinical laboratory (hematology, clinical chemistry, coagulation, and urinalysis), and electrocardiograms (ECGs) on enrolled patients are monitored. Additionally, available PK/PD data and indicators of clinical activity (e.g., changes from baseline in hemoglobin (Hb)) are assessed.

### Pharmacokinetic and Pharmacodynamic Assessments

Pharmacokinetic assessments include serial blood sampling for determination of concentration-time profiles of Compound 1 and are conducted, e.g., following the first dose and the morning Day 15 dose. For example, additional trough levels of Compound 1 are obtained. Compound 1 is analyzed using assays to determine concentrations in plasma. Pharmacokinetic parameters on Day 1 and Day 15 are computed using standard non-compartmental methods based on observed plasma Compound 1 concentrations.

Pharmacodynamic assessments include serial blood sampling for determination of levels of ATP and 2,3-DPG. Serial blood sampling for determination of levels of ATP and, 2,3-DPG is conducted, e.g., following the first dose and the morning Day 15 dose, and additional trough levels of ATP and 2,3-DPG will be obtained. ATP and 2,3-DPG are analyzed using assays to determine concentrations in whole blood. Pharmacodynamic parameters on Day 1 and Day 15 are computed based on observed whole blood ATP and 2,3-DPG concentrations.

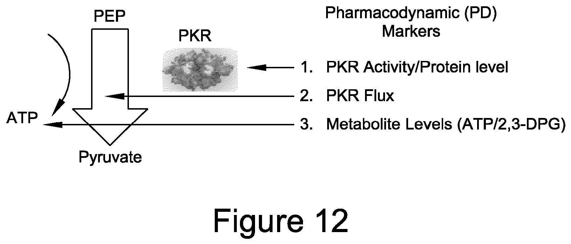

In some cases, assessments include determination of PKR activity, PKR protein, and glycolytic flux assays. Blood samples are evaluated for PKR activity in RBCs, and assessment of glycolytic flux in whole blood is performed through *ex-vivo* labeling with ¹³C-glucose. Blood is also evaluated for total PKR protein levels. Levels of additional metabolites are also assessed in blood samples to further elucidate the mechanism and effects of PKR activation by Compound 1. Exemplary PD markers are shown in Figure 12. The PKR enzyme catalyzes the PEP-to-pyruvate reaction, with concomitant formation of ATP. Binding of Compound 1 to the PKR tetramer can be assessed through an ex-vivo biochemical assay of cell lysates from Compound 1 treated patients. PKR protein levels in whole blood are assessed through Western blotting or quantitative ELISA (or other similar assay).

The PKR Flux assay measures the change in carbon flow from glucose through the PKR reaction to pyruvate after Compound 1 treatment. This is distinct from the target engagement measured by the PKR activity assay because it is conducted in intact cells and thus a more direct and functional measure of pathway activity. The PKR Flux assay is performed by incubating freshly drawn patient blood at 37 degrees C with ¹³C6-labeled glucose. Aliquots from the incubation reaction are taken over time and flash frozen. Subsequent analysis by mass spectrometry reveals the rate of label incorporation into glycolytic intermediates including DHAP, 2,3-DPG, 3-PG, and PEP. The data are fitted by mathematical modeling to quantitate the carbon flow through the PKR reaction. An increase in carbon flow through the PKR reaction indicates efficacy of Compound 1. Compound 1 target engagement and stimulation of glycolytic pathway activity has been shown in preclinical models and healthy volunteer clinical studies to result in accumulation of ATP and depletion of the upstream metabolite 2,3-DPG. Therefore, an increase in ATP levels and/or a decrease in 2,3-DPG levels indicates efficacy of Compound 1. Levels of these metabolites can be measured by mass spectrometry from frozen whole blood samples.

In some cases, exposure-response analysis is performed to evaluate the relationship of Compound 1 exposure and PD effects with changes in indicators of clinical activity (e.g., changes in Hb levels).

Additional or alternative data/observations other than those listed above may be reviewed. Based on the reviews, one or more of the following steps may be implemented:
- Add 1 new dose arm (Arm 3) to enroll up to 25 patients at a dose to be determined; the dose for Arm 3 may be lower or higher than Arm 1 and Arm 2 doses, but will not exceed 360 mg q12h; and the dose regimen may be less frequent than q12h.

The foregoing description and drawings are by way of example only.

## Claims

1. A pharmaceutical composition comprising Compound 1 or a pharmaceutically acceptable salt or hydrate thereof in an orally acceptable dosage form for use in a method for treating thalassemia in a subject in need thereof, comprising orally administering to the subject the pharmaceutical composition comprising Compound 1 or a pharmaceutically acceptable salt or hydrate thereof at a dose of about 60 mg to about 200 mg of Compound 1 once or twice daily to thereby treat thalassemia in the subject, wherein Compound 1 is N-(4-(4-(cyclopropylmethyl)piperazine-1-carbonyl)phenyl)quinoline-8-sulfonamide.

2. The pharmaceutical composition comprising Compound 1 or a pharmaceutically acceptable salt or hydrate thereof in an orally acceptable dosage form for use of claim 1, wherein the method comprises orally administering to the subject the pharmaceutical composition comprising Compound 1 or a pharmaceutically acceptable salt or hydrate thereof at a dose of about 100 mg of Compound 1 once or twice daily.

3. The pharmaceutical composition comprising Compound 1 or
a pharmaceutically acceptable salt or hydrate thereof in an orally acceptable dosage form for use of claim 1 or 2, wherein the thalassemia is beta-thalassemia.

4. A pharmaceutical composition comprising Compound 1 or
a pharmaceutically acceptable salt or hydrate thereof in an orally acceptable dosage form for use in a method for treating sickle cell anemia in a subject in need thereof, comprising orally administering to the subject the pharmaceutical composition comprising Compound 1 or
a pharmaceutically acceptable salt or hydrate thereof at a dose of about 60 mg to about 200 mg of Compound 1 once or twice daily to thereby treat sickle cell anemia in the subject, wherein Compound 1 is N-(4-(4-(cyclopropylmethyl)piperazine-1-carbonyl)phenyl)quinoline-8-sulfonamide.

5. The pharmaceutical composition comprising Compound 1 or
a pharmaceutically acceptable salt or hydrate thereof in an orally acceptable dosage form for use of claim 4, wherein the method comprises orally administering to the subject the pharmaceutical composition comprising a Compound 1 or a pharmaceutically acceptable salt or hydrate thereof at a dose of about 100 mg of Compound 1 once or twice daily.

6. The pharmaceutical composition comprising Compound 1 or
a pharmaceutically acceptable salt or hydrate thereof in an orally acceptable dosage form for use of any one of claims 1 to 5, wherein the method comprises administering the pharmaceutical composition comprising Compound 1 or a pharmaceutically acceptable salt or hydrate thereof twice daily.

7. The pharmaceutical composition comprising Compound 1 or
a pharmaceutically acceptable salt or hydrate thereof in an orally acceptable dosage form for use of any one of claims 1 to 5, wherein the method comprises administering to the subject the pharmaceutical composition comprising Compound 1 or a pharmaceutically acceptable salt or hydrate thereof at a dose of about 100 mg of Compound 1 twice daily.

8. The pharmaceutical composition comprising Compound 1 or a pharmaceutically acceptable salt or hydrate thereof for use of any one of claims 1 to 5, wherein the method comprises administering to the subject the pharmaceutical composition comprising Compound 1 or
a pharmaceutically acceptable salt or hydrate thereof at a dose of about 200 mg of Compound 1 about every 24 hours.

## Patentansprüche

1. Pharmazeutische Zusammensetzung umfassend Verbindung 1 oder ein pharmazeutisch verträgliches Salz oder Hydrat davon in einer oral verträglichen Darreichungsform zur Verwendung in einem Verfahren zur Behandlung von Thalassämie bei einem Subjekt, das eine solche Behandlung benötigt, wobei dem Subjekt die pharmazeutische Zusammensetzung umfassend Verbindung 1 oder ein pharmazeutisch verträgliches Salz oder Hydrat davon oral in einer Dosis von etwa 60 mg bis etwa 200 mg der Verbindung 1 ein- oder zweimal täglich verabreicht wird, um dabei die Thalassämie bei dem Subjekt zu behandeln, wobei die Verbindung 1 N-(4-(4-(Cyclopropylmethyl)piperazin-1-carbonyl)phenyl)chinolin-8-sulfonamid ist.

2. Pharmazeutische Zusammensetzung umfassend Verbindung 1 oder ein pharmazeutisch verträgliches Salz oder Hydrat davon in einer oral verträglichen Darreichungsform zur Verwendung nach Anspruch 1, wobei das Verfahren die orale Verabreichung der pharmazeutischen Zusammensetzung umfassend die Verbindung 1 oder ein pharmazeutisch verträgliches Salz oder Hydrat davon in einer Dosis von etwa 100 mg der Verbindung 1 ein- oder zweimal täglich umfasst.

3. Pharmazeutische Zusammensetzung umfassend Verbindung 1 oder ein pharmazeutisch verträgliches Salz oder Hydrat davon in einer oral verträglichen Darreichungsform zur Verwendung nach Anspruch 1 oder 2, wobei die Thalassämie eine Beta-Thalassämie ist.

4. Pharmazeutische Zusammensetzung umfassend Verbindung 1 oder ein pharmazeutisch verträgliches Salz oder Hydrat davon in einer oral verträglichen Darreichungsform zur Verwendung in einem Verfahren zur Behandlung von Sichelzellenanämie bei einem Subjekt, das eine solche Behandlung benötigt, wobei dem Subjekt die pharmazeutische Zusammensetzung umfassend Verbindung 1 oder ein pharmazeutisch verträgliches Salz oder Hydrat davon oral in einer Dosis von etwa 60 mg bis etwa 200 mg der Verbindung 1 ein- oder zweimal täglich verabreicht wird, um dabei die Sichelzellenanämie bei dem Subjekt zu behandeln, wobei die Verbindung 1 N-(4-(4-(Cyclopropylmethyl)piperazin-1-carbonyl)phenyl)chinolin-8-sulfonamid ist.

5. Pharmazeutische Zusammensetzung umfassend Verbindung 1 oder ein pharmazeutisch verträgliches Salz oder Hydrat davon in einer oral verträglichen Darreichungsform zur Verwendung nach Anspruch 4, wobei das Verfahren die orale Verabreichung der pharmazeutischen Zusammensetzung umfassend Verbindung 1 oder ein pharmazeutisch verträgliches Salz oder Hydrat davon in einer Dosis von etwa 100 mg der Verbindung 1 ein- oder zweimal täglich umfasst.

6. Pharmazeutische Zusammensetzung umfassend Verbindung 1 oder ein pharmazeutisch verträgliches Salz oder Hydrat davon in einer oral verträglichen Darreichungsform zur Verwendung nach einem der Ansprüche 1 bis 5, wobei das Verfahren die orale Verabreichung der pharmazeutischen Zusammensetzung umfassend Verbindung 1 oder ein pharmazeutisch verträgliches Salz oder Hydrat davon zweimal täglich umfasst.

7. Pharmazeutische Zusammensetzung umfassend Verbindung 1 oder ein pharmazeutisch verträgliches Salz oder Hydrat davon in einer oral verträglichen Darreichungsform zur Verwendung nach einem der Ansprüche 1 bis 5, wobei das Verfahren die orale Verabreichung der pharmazeutischen Zusammensetzung umfassend Verbindung 1 oder ein pharmazeutisch verträgliches Salz oder Hydrat davon in einer Dosis von etwa 100 mg der Verbindung 1 zweimal täglich umfasst.

8. Pharmazeutische Zusammensetzung umfassend Verbindung 1 oder ein pharmazeutisch verträgliches Salz oder Hydrat davon in einer oral verträglichen Darreichungsform zur Verwendung nach einem der Ansprüche 1 bis 5, wobei das Verfahren die orale Verabreichung der pharmazeutischen Zusammensetzung umfassend Verbindung 1 oder ein pharmazeutisch verträgliches Salz oder Hydrat davon in einer Dosis von etwa 200 mg der Verbindung 1 etwa alle 24 Stunden umfasst.

## Revendications

1. Composition pharmaceutique comprenant le Composé 1 ou un sel ou hydrate pharmaceutiquement acceptable de celui-ci sous une forme posologique acceptable par voie orale, pour utilisation dans une méthode de traitement de la thalassémie chez un sujet qui en a besoin, comprenant l'administration par voie orale au sujet de la composition pharmaceutique comprenant le Composé 1 ou un sel ou hydrate pharmaceutiquement acceptable de celui-ci à une dose d'environ 60 mg à environ 200 mg de Composé 1 une ou deux fois par jour pour traiter ainsi la thalassémie chez le sujet, dans laquelle le Composé 1 est la N-(4-(4-(cyclopropylméthyl)pipérazine-1-carbonyl)phényl)quinoléine-8-sulfonamide.

2. La composition pharmaceutique comprenant le Composé 1 ou un sel ou hydrate pharmaceutiquement acceptable de celui-ci sous une forme posologique acceptable par voie orale pour l'utilisation selon la revendication 1, dans laquelle la méthode comprend l'administration par voie orale au sujet de la composition pharmaceutique comprenant le Composé 1 ou un sel ou hydrate pharmaceutiquement acceptable de celui-ci à une dose d'environ 100 mg de Composé 1 une ou deux fois par jour.

3. La composition pharmaceutique comprenant le Composé 1 ou un sel ou hydrate pharmaceutiquement acceptable de celui-ci sous une forme posologique acceptable par voie orale pour l'utilisation selon la revendication 1 ou 2, dans laquelle la thalassémie est une bêta-thalassémie.

4. Composition pharmaceutique comprenant le Composé 1 ou un sel ou hydrate pharmaceutiquement acceptable de celui-ci sous une forme posologique acceptable par voie orale pour utilisation dans une méthode de traitement de la drépanocytose chez un sujet qui en a besoin, comprenant l'administration par voie orale au sujet de la composition pharmaceutique comprenant le Composé 1 ou un sel ou hydrate pharmaceutiquement acceptable de celui-ci à une dose d'environ 60 mg à environ 200 mg de Composé 1 une ou deux fois par jour pour traiter ainsi
la drépanocytose chez le sujet, dans laquelle le Composé 1 est la N-(4-(4-(cyclopropylméthyl)pipérazine-1-carbonyl)phényl)quinoléine-8-sulfonamide.

5. La composition pharmaceutique comprenant le Composé 1 ou un sel ou hydrate pharmaceutiquement acceptable de celui-ci sous une forme posologique acceptable par voie orale pour l'utilisation selon la revendication 4, dans laquelle la méthode comprend l'administration par voie orale au sujet de la composition pharmaceutique comprenant un Composé 1 ou un sel ou hydrate pharmaceutiquement acceptable de celui-ci à une dose d'environ 100 mg de Composé 1 une ou deux fois par jour.

6. La composition pharmaceutique comprenant le Composé 1 ou un sel ou hydrate pharmaceutiquement acceptable de celui-ci sous une forme posologique acceptable par voie orale pour l'utilisation selon l'une quelconque des revendications 1 à 5, dans laquelle la méthode comprend l'administration de la composition pharmaceutique comprenant le Composé 1 ou un sel ou hydrate pharmaceutiquement acceptable de celui-ci deux fois par jour.

7. La composition pharmaceutique comprenant le Composé 1 ou un sel ou hydrate pharmaceutiquement acceptable de celui-ci sous une forme posologique acceptable par voie orale pour l'utilisation selon l'une quelconque des revendications 1 à 5, dans laquelle la méthode comprend l'administration au sujet de la composition pharmaceutique comprenant le Composé 1 ou un sel ou hydrate pharmaceutiquement acceptable de celui-ci à une dose d'environ 100 mg de Composé 1 deux fois par jour.

8. La composition pharmaceutique comprenant le Composé 1 ou un sel ou hydrate pharmaceutiquement acceptable de celui-ci pour l'utilisation selon l'une quelconque des revendications 1 à 5, dans laquelle la méthode comprend l'administration au sujet de la composition pharmaceutique comprenant le Composé 1 ou un sel ou hydrate pharmaceutiquement acceptable de celui-ci à une dose d'environ 200 mg de Composé 1 environ toutes les 24 heures.
